# EUROPEAN PATENT SPECIFICATION

(11) **EP 2 707 482 B1**
(45) Date of publication and mention of the grant of the patent: **09.09.2015**
(21) Application number: 12720503.7
(22) Date of filing: 11.05.2012
(51) Int. Cl.: C12N 11/16, C07K 19/00, C12P 5/00

(54) **WHOLE CELL BIOCATALYST COMPRISING A PRENYLTRANSFERASE**
GANZZELLEN-BIOKATALYSATOR MIT EINER PRENYLTRANSFERASE
BIOCATALYSEUR POUR CELLULES ENTIÈRES COMPRENANT UNE PRÉNYLTRANSFÉRASE

(30) Priority: 12.05.2011 EP 11165903
(43) Date of publication of application: 19.03.2014
(73) Proprietor: Autodisplay Biotech GmbH, 40225 Düsseldorf (DE)
(72) Inventor: JOSE, Joachim, 40589 Düsseldorf (DE); MAAS, Ruth Maria, 40589 Düsseldorf (DE); KRANEN, Eva Elisabeth, 40219 Düsseldorf (DE); STEFFAN, Nicola, 53123 Bonn (DE); LI, Shu-Ming, 35043 Marburg (DE)
(74) Representative: Weickmann & Weickmann
(86) International application number: PCT/EP2012/058782
(87) International publication number: WO 2012/152923

(56) References cited:
- WO-A2-02/070645
- JOSE J ET AL: "The autodisplay story, from discovery to biotechnical and biomedical applications", MICROBIOLOGY AND MOLECULAR BIOLOGY REVIEWS, AMERICAN SOCIETY FOR MICROBIOLOGY, US, vol. 71, no. 4, 1 December 2007 (2007-12-01), pages 600-619, XP002577399, ISSN: 1092-2172, DOI: 10.1128/MMBR.00011-07
- SAMUELSON P ET AL: "Display of proteins on bacteria", JOURNAL OF BIOTECHNOLOGY, ELSEVIER SCIENCE PUBLISHERS, AMSTERDAM, NL, vol. 96, no. 2, 26 June 2002 (2002-06-26), pages 129-154, XP002321735, ISSN: 0168-1656, DOI: 10.1016/S0168-1656(02)00043-3
- CHRISTIAN DETZEL ET AL: "Autodisplay of Nitrilase from Alcaligenes faecalis in E coli Yields a Whole Cell Biocatalyst for the Synthesis of Enantiomerically Pure (R)-Mandelic Acid", CHEMCATCHEM : HETEROGENEOUS & HOMOGENEOUS & BIO-CATALYSIS, WEINHEIM : WILEY-VCH, DE, no. 3, 11 April 2011 (2011-04-11), pages 719-725, XP002636113, ISSN: 1867-3880, DOI: 10.1002/CCTC.201000382 [retrieved on 2011-02-04]
- YAMADA Y ET AL: "Efficient in vitro synthesis of cis-polyisoprenes using a thermostable cis-prenyltransferase from a hyperthermophilic archaeon Thermococcus kodakaraensis", JOURNAL OF BIOTECHNOLOGY, ELSEVIER SCIENCE PUBLISHERS, AMSTERDAM, NL, vol. 143, no. 2, 20 August 2009 (2009-08-20), pages 151-156, XP026467238, ISSN: 0168-1656, DOI: 10.1016/J.JBIOTEC.2009.06.026 [retrieved on 2009-07-05]
- LIANG PO-HUANG ET AL: "Structure, mechanism and function of prenyltransferases.", EUROPEAN JOURNAL OF BIOCHEMISTRY / FEBS JUL 2002 LNKD- PUBMED:12135472, vol. 269, no. 14, July 2002 (2002-07), pages 3339-3354, XP002678416, ISSN: 0014-2956
- EVA KRANEN ET AL: "Development of a Whole Cell Biocatalyst for the Efficient Prenylation of Indole Derivatives by Autodisplay of the Aromatic Prenyltransferase FgaPT2", CHEMCATCHEM, vol. 3, no. 7, 30 May 2011 (2011-05-30), pages 1200-1207, XP55030733, ISSN: 1867-3880, DOI: 10.1002/cctc.201000429

### SEQUENCE LISTING

<110> Autodisplay Biotech GmbH
<120> Whole cell biocatalyst for the prenylation of indole derivatives
<130> 497335PWO
<160> 8
<170> PatentIn version 3.5
<210> 1
   <211> 1389
   <212> DNA
   <213> Aspergillus fumigatus
<220>
   <221> misc_feature
   <222> (1)..(1389)
   <223> FgaPT2
<400> 1
   ctcgagatga aggcagccaa tgcctccagt gcggaggcct atcgagttct tagtcgcgcc      60
   tttagattcg ataatgaaga tcagaagctg tggtggcaca gcactgcccc gatgtttgca     120
   aaaatgctgg aaactgccaa ctacaccaca ccttgtcagt atcaatacct catcacctat     180
   aaggagtgcg taattcccag tctcggatgc tatccgacca acagcgcccc ccgctggttg     240
   agcatcctca ctcgatacgg cactccgttc gaattgagcc taaattgctc taattcaata     300
   gtgagataca cattcgagcc gatcaatcaa cataccggaa cagataaaga cccattcaat     360
   acgcacgcca tctgggagag cctgcagcac ctgcttccac tggagaagag cattgatctg     420
   gagtggttcc gccacttcaa gcacgatctc accctcaaca gtgaagaatc tgcttttctg     480
   gctcataatg atcgcctcgt gggcggcact atcaggacgc agaacaagct cgcgctcgat     540
   ctgaaggatg gccgctttgc acttaagacg tacatatacc cggctctcaa agctgtcgtc     600
   accggcaaga caattcatga gttggtcttt ggctcagtcc gccggctggc agtgagggag     660
   ccccgaatct tgcccccact caacatgctg gaggaataca tccgatcacg cggttccaag     720
   agcactgcca gtccccgcct agtgtcctgt gatctgacca gtcctgccaa gtcgagaatc     780
   aagatctacc tgctggagca gatggtttca ctagaagcca tggaggacct gtggactctg     840
   ggcggacggc gccgagacgc ttccacttta gaggggctct ctctggtgcg tgagctttgg     900
   gatctgatcc aactgtcgcc gggattgaag tcctatccgg cgccgtatct gcctctcggg     960
   gttatcccag acgagaggct gccgcttatg gccaatttca ccctgcacca gaatgacccg    1020
   gtcccagagc cgcaagtata tttcacaacc ttcggcatga acgacatggc ggtggcggat    1080
   gccctgacga cgttcttcga gcgccggggt tggagtgaaa tggcccgcac ctacgaaact    1140
   actttgaagt cgtactaccc ccatgcggat catgacaaac ttaactacct ccacgcctac    1200
   atatccttct cctacaggga ccgtacccct tatctgagtg tctatcttca atccttcgag    1260
   acaggggact gggcagttgc aaacttatcc gaatcaaagg tcaagtgtca ggatgcggcc    1320
   tgtcaaccca cagctttacc tccagatctg tcaaagacag gggtatatta ttccggtctc    1380
   cacggtacc                                                            1389
<210> 2
   <211> 463
   <212> PRT
   <213> Aspergillus fumigatus
<220>
   <221> MISC_FEATURE
   <223> amino acid sequence of the fgaPT2-PCR-product
<400> 2
   Leu Glu Met Lys Ala Ala Asn Ala Ser Ser Ala Glu Ala Tyr Arg Val
   1               5                   10                  15
   Leu Ser Arg Ala Phe Arg Phe Asp Asn Glu Asp Gln Lys Leu Trp Trp
               20                  25                  30
   His Ser Thr Ala Pro Met Phe Ala Lys Met Leu Glu Thr Ala Asn Tyr
           35                  40                  45
   Thr Thr Pro Cys Gln Tyr Gln Tyr Leu Ile Thr Tyr Lys Glu Cys Val
       50                  55                  60
   Ile Pro Ser Leu Gly Cys Tyr Pro Thr Asn Ser Ala Pro Arg Trp Leu
   65                  70                  75                  80
   Ser Ile Leu Thr Arg Tyr Gly Thr Pro Phe Glu Leu Ser Leu Asn Cys
                   85                  90                  95
   Ser Asn Ser Ile Val Arg Tyr Thr Phe Glu Pro Ile Asn Gln His Thr
               100                 105                 110
   Gly Thr Asp Lys Asp Pro Phe Asn Thr His Ala Ile Trp Glu Ser Leu
           115                 120                 125
   Gln His Leu Leu Pro Leu Glu Lys Ser Ile Asp Leu Glu Trp Phe Arg
       130                 135                 140
   His Phe Lys His Asp Leu Thr Leu Asn Ser Glu Glu Ser Ala Phe Leu
   145                 150                 155                 160
   Ala His Asn Asp Arg Leu Val Gly Gly Thr Ile Arg Thr Gln Asn Lys
               165                 170                 175
   Leu Ala Leu Asp Leu Lys Asp Gly Arg Phe Ala Leu Lys Thr Tyr Ile
           180                 185                 190
   Tyr Pro Ala Leu Lys Ala Val Val Thr Gly Lys Thr Ile His Glu Leu
       195                 200                 205
   Val Phe Gly Ser Val Arg Arg Leu Ala Val Arg Glu Pro Arg Ile Leu
   210                 215                 220
   Pro Pro Leu Asn Met Leu Glu Glu Tyr Ile Arg Ser Arg Gly Ser Lys
   225                 230                 235                 240
   Ser Thr Ala Ser Pro Arg Leu Val Ser Cys Asp Leu Thr Ser Pro Ala
                   245                 250                 255
   Lys Ser Arg Ile Lys Ile Tyr Leu Leu Glu Gln Met Val Ser Leu Glu
               260                 265                 270
   Ala Met Glu Asp Leu Trp Thr Leu Gly Gly Arg Arg Arg Asp Ala Ser
           275                 280                 285
   Thr Leu Glu Gly Leu Ser Leu Val Arg Glu Leu Trp Asp Leu Ile Gln
       290                 295                 300
   Leu Ser Pro Gly Leu Lys Ser Tyr Pro Ala Pro Tyr Leu Pro Leu Gly
   305                 310                 315                 320
   Val Ile Pro Asp Glu Arg Leu Pro Leu Met Ala Asn Phe Thr Leu His
                   325                 330                 335
   Gln Asn Asp Pro Val Pro Glu Pro Gln Val Tyr Phe Thr Thr Phe Gly
               340                 345                 350
   Met Asn Asp Met Ala Val Ala Asp Ala Leu Thr Thr Phe Phe Glu Arg
           355                 360                 365
   Arg Gly Trp Ser Glu Met Ala Arg Thr Tyr Glu Thr Thr Leu Lys Ser
       370                 375                 380
   Tyr Tyr Pro His Ala Asp His Asp Lys Leu Asn Tyr Leu His Ala Tyr
   385                 390                 395                 400
   Ile Ser Phe Ser Tyr Arg Asp Arg Thr Pro Tyr Leu Ser Val Tyr Leu
                   405                 410                 415
   Gln Ser Phe Glu Thr Gly Asp Trp Ala Val Ala Asn Leu Ser Glu Ser
   420 425 430
   Lys Val Lys Cys Gln Asp Ala Ala Cys Gln Pro Thr Ala Leu Pro Pro
   435 440 445
   Asp Leu Ser Lys Thr Gly Val Tyr Tyr Ser Gly Leu His Gly Thr
   450 455 460
<210> 3
   <211> 66
   <212> DNA
   <213> Artificial Sequence
<220>
   <223> surrounding of the fusion sites
<220>
   <221> misc_feature
   <223> Surrounding of the fusion sites
<400> 3
   tatgcacatg gaacacctca aaatattact gatttgctcg agatgaaggc agccaatgcc 60
   tccagt 66
<210> 4
   <211> 22
   <212> PRT
   <213> Artificial Sequence
<220>
   <223> surrounding of the fusion sites
<220>
   <221> MISC_FEATURE
   <223> Surrounding of the fusion sites
<400> 4
   Tyr Ala His Gly Thr Pro Gln Asn Ile Thr Asp Leu Leu Glu Met Lys
   1 5 10 15
   Ala Ala Asn Ala Ser Ser
   20
<210> 5
   <211> 29
   <212> DNA
   <213> Artificial Sequence
<220>
   <223> Primer EK003
<400> 5
   ctgctcgaga tgaaggcagc caatgcctc 29
<210> 6
   <211> 30
   <212> DNA
   <213> Artificial Sequence
<220>
   <223> Primer EK004
<400> 6
   ggggtaccgt ggagaccgga ataatatacc 30
<210> 7
   <211> 57
   <212> DNA
   <213> Artificial Sequence
<220>
   <223> surrounding of the fusion sites
<400> 7
   tattccggtc tccacggtac ccttaatcct acaaaagaaa gtgcaggtaa tactctt 57
<210> 8
   <211> 19
   <212> PRT
   <213> Artificial Sequence
<220>
   <223> Surrounding of the fusion sites
<400> 8
   Tyr Ser Gly Leu His Gly Thr Leu Asn Pro Thr Lys Glu Ser Ala Gly
   1               5                   10                  15
   Asn Leu Thr

## Description

The present invention relates to a method for functionally displaying a recombinant prenyltransferase on the surface of a host cell.

Over the past 30 years, it has become clear that enzymes hold great potential for industry. They are most remarkable biomolecules because of their extraordinary specificity and catalytic power. The specificity and (enantio- and regio-)selectivity of certain enzymatic transformations makes them attractive for the production of fine chemicals and pharmaceutical intermediates. To date, more than 500 products are manufactured by enzymes. Well-known examples are ephedrine, aspartame and amoxicillin.

Prenyltransferases catalyze the transfer of an activated isoprenoid moiety derived from allylic isoprenyl diphosphates (e.g. dimethylallyl diphosphate (C₅), geranyl diphosphate (C₁₀) or farnesyl diphosphate (C₁₅)) onto a nucleophilic acceptor molecule. They are subdivided into three groups being isoprenylpyrophosphate synthases, protein prenyltransferases and aromatic prenyltransferases [1].

Isoprenylpyrophosphate synthases catalyze the chain elongation of allylic isoprenyl pyrophosphate substrates with isopentenyl pyrophosphate. The resulting isoprenoids are precursors for a large variety of natural products e.g.terpenes and steroids [2-3]. Yamada et al. (J. Biotechnology, 2009, 143:151-156) discloses a cis-prenyltransferase from Thermococcus kodakaraensis.

Protein prenyltransferases catalyze the transfer of a prenyl moiety (C₁₅ or C₂₀) onto protein or peptide substrates. This posttranslational modification is vital for the function of signal transducing proteins like Ras or other G-proteins [4-5].

Aromatic prenyltransferases attach isoprenoid moieties to aromatic acceptor molecules. Based upon this prenylation reaction, which is often the rate limiting step in the respective pathway, many different primary and secondary metabolites are generated in plants, fungi and bacteria [6-7]. It has been shown that this prenylation step enhances the pharmacological activities of some flavonoids compared to their non-prenylated precursors [8-9]. This effect is presumably caused by an increase in the affinity for biological membranes and improved interactions with cellular targets [10].

Another class of prenylated compounds from *Aspergillus fumigatus*, tryptophan derived cyclic dipeptides, exhibited enormous cytotoxic effects [11]. Prenylated products also function as precursors or intermediates for secondary metabolites [7]. For example the first step in ergot alkaloid synthesis is the prenylation of tryptophan [12].

Because of their low substrate specificity, aromatic prenyltransferases can be regarded as an interesting biocatalyst for the synthesis of new pharmacologically active compounds or intermediates [13].

FgaPT2 is an aromatic prenyltransferase from *Aspergillus fumigatus*. It is a soluble homodimeric protein with a subunit size of 52 kDa [14]. It catalyzes the first step in ergot alkaloid synthesis in *A. fumigatus* by transferring an activated dimethylallyl group to tryptophan and therefore can be more specifically described as a dimethylallyltryptophan synthase [12].

The gene encoding for the dimethylallyltryptophan synthase FgaPT2 was originally identified in the genome sequence of *A. fumigatus*, heterologously expressed in *Saccharomyces cerevisiae*, and therein isolated and characterized [15]. It has also been expressed in *Escherichia coli* thus far and prenylation of different indole derivatives was investigated [16]. Since most of the aromatic prenyltransferases show a low substrate specificity [7, 13], FgaPT2 also exhibits substrate promiscuity and accepts 17 different indole derivatives [16]. A recent study suggests that also tryptophan containing dipeptides are accepted as substrates by FgaPT2 [17]. It thus represents a promising biocatalytic tool in enzymatic synthesis of active indole or indole-dipeptide derivatives [7, 13].

One way to establish an efficient biocatalytic process is to use whole cell biocatalysts constructed using surface display. Enzymes displayed on the cell surface of e. g. gram negative bacteria are immobilized and thus more stable [18]. Presentation of the enzyme on the surface enables the direct contact between substrate and enzyme, making the membrane passage of the substrate unnecessary. Coupling the enzyme to an insoluble and rather large particle like an *E*. *coli* cell, furthermore allows the time- and cost-effective recovery of the biocatalyst. Maintaining the biocatalyst is achieved by simply cultivating the *E*. *coli* cells.

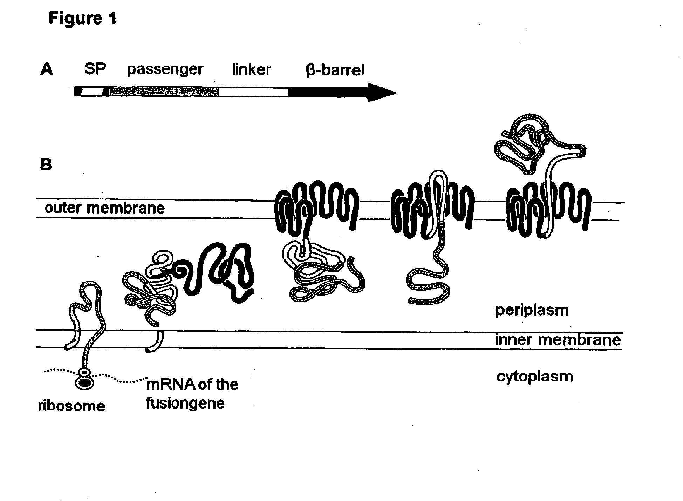

A proven method for the development of surface engineered whole cell biocatalysts is Autodisplay. The Autodisplay system allows the display of different types of proteins on the surface of *E*. *coli* [19]. These proteins are integrated as a passenger in a precursor protein consisting of a signal peptide, which facilitates the transport across the inner membrane, and a C-terminal β-barrel structure, which enables the translocation across the outer membrane. The passenger and β-barrel are connected by a linker peptide, assuring the complete display of the protein of interest (Figure 1) [20]. This translocation subunit consisting of linker and β-barrel subunit is derived from the AIDA-1 (adhesine involved in diffuse adherence) autotransporter protein of *E*. *coli* [21]. Since the β-barrels are motile in the outer membrane, multimeric proteins displayed with this technique show the spontaneous aggregation of passengers to form dimers or even higher ordered oligomers [22-24]. Examples of functional enzymes expressed using Autodisplay for functional whole cell biocatalysis include an esterase, a sorbitol dehydrogenase and a nitrilase [23-25]. WO 02/07065 A2 discloses the functional display of adrenodoxin dimers by the AIDA-I autotransporter on the surface of *E*. *coli*.

The application of surface display systems such as Autodisplay for the development of whole cell biocatalysts comprises many advantages compared with conventional whole cell biocatalysts, where the enzyme of interest resides within the cell cytoplasm. Firstly, transport across membranes is not necessary for substrates or products [18], secondly, product and catalyst can be easily recovered by removing the cells from the reaction. Thirdly, it is possible to avoid the formation of by-products or metabolism of the substrate or desired product inside the cell, which is often a problem in whole cell biocatalysis [31-32].

In the present invention, it has surprisingly been found that stability and efficiency of a prenyltransferase can be improved if the prenyltransferase is stably displayed on the surface of a host cell, compared with the purified enzyme.

A first aspect of the present invention is a method for displaying a recombinant prenyltransferase on the surface of a host cell, said method comprising the steps:
(a) providing a host cell transformed with a nucleic acid fusion operatively linked with an expression control sequence, said nucleic acid fusion comprising:
   (i) a portion encoding a signal peptide,
   (ii) a portion encoding the recombinant prenyltransferase to be displayed,
   (iii) optionally a portion encoding a protease recognition site,
   (iv) a portion encoding a transmembrane linker, and
   (v) a portion encoding the transporter domain of an autotransporter, and
(b) culturing the host cell under conditions wherein the nucleic acid fusion is expressed and the expression product comprising the recombinant prenyltransferase is displayed on the surface of the host cell.

The expression product is also referred herein as "polypeptide of the present invention".

In the context of the present invention, "prenylation" is the introduction of an isoprene moiety or/and an isoprenoid moiety. One or more isoprene moieties or/and isoprenoid moieties may be introduced. Prenylation can be catalyzed by prenyltransferases capable of transferring an activated isoprenoid moiety onto a nucleophilic acceptor molecule.

Isoprene refers to 2-methyl-1,3-butadiene.

"Isoprenoid moiety" or "isoprenoid", as used herein, refers to a condensation product of two or more isoprene units. Biosynthesis of isoprenoids starts with the isomerization of isopentenyl pyrophosphate (IPP) to dimethylallyl pyrophosphate (DMAPP, also termed DMAP). These two C₅ units can condense to form geranyl pyrophosphate (C₁₀) which can condense with another isopentenyl pyrophosphate to form farnesyl pyrophosphate (C₁₅) (see, for example, Stryer, Biochemistry, 2nd ed. 1981). In further steps, a various number of isopentenyl pyrophosphate molecules can be added. Examples of isoprenoids include terpens, terpenoids and steroids.

The term "allylic isoprenyl diphosphate", "allylic isoprenyl pyrophosphate", "activated allylic isoprenyl diphosphate", or "activated allylic isoprenyl pyrophosphate" refers to an isoprenoid compound comprising an allyl function and a pyrophosphate group, wherein condensation of a further isopentenyl pyrophosphate molecule can take place at the carbon atom where the pyrophosphate group is bound. "Allylic isoprenyl diphosphate" and "allylic isoprenyl pyrophosphate" are terms commonly used in the field of isoprenoid biosynthesis.

Examples of allylic isoprenyl diphosphates include dimethylallyl diphosphate (C₅), geranyl diphosphate (C₁₀), farnesyl diphosphate (C₁₅), and geranylgeranyl diphosphate (C₂₀).

"Activation" or "activated", as used herein, refers to the presence of a pyrophosphate group (also termed herein diphosphate group) in an isoprenoid compound.

In the present invention, the prenyltransferase can be selected from isoprenylpyrophosphate synthases, protein prenyltransferases, and aromatic prenyltransferases.

In particular, the prenyltransferase can be selected from dimethylallyltranstransferase (EC 2.5.1.1), (2E, 6E)-farnesyl diphosphate synthase (EC 2.5.1.10), rubber cis-polyprenylcistransferase (EC 2.5.1.20), squalene synthase (EC 2.5.1.21), dimethylallylcistransferase (EC 2.5.1.28), farnesyltranstransferase (EC 2.5.1.29), heptaprenyl diphosphate synthase (EC 2.5.1.30), ditrans,polycis-Undecaprenyl diphosphate synthase (EC 2.5.1.31), phytoene synthase (EC 2.5.1.32), 4-dimethylallyltryptophan synthase (EC 2.5.1.34), aspulvinone dimethylallyltransferase (EC 2.5.1.35), trihydroxypterocarpan dimethylallyltransferase (EC 2.5.1.36), 4-hydroxybenzoate polyprenyltransferase (EC 2.5.1.39), phosphoglycerol geranylgeranyltransferase (EC 2.5.1.41), geranylgeranylglycerol-phosphate-geranylgeranyltransferase (EC 2.5.1.42), protein farnesyltransferase (EC 2.5.1.58), protein geranylgeranyltransferase Typ I (GGTase I) (EC 2.5.1.59), protein geranylgeranyltransferase Typ II (RabGGTase) (EC 2.5.1.60), chrysanthemyl diphosphate synthase (EC 2.5.1.67), (2Z,6E)-farnesyl diphosphate synthase (EC 2.5.1.68), lavandulyl diphosphate synthase (EC 2.5.1.69), naringenin-8-dimethylallyltransferase (EC 2.5.1.70), leachianon-G2"-dimethylallyltransferase (EC 2.5.1.71), 1,4-dihydroxy-2-naphtoat polyprenyltransferase (EC 2.5.1.74), 7-dimethylallyltryptophan synthase (EC 2.5.1.80), geranylfarnesyl diphosphate synthase (EC 2.5.1.81), hexaprenyl diphosphate synthase (EC 2.5.1.82), hexaprenyl diphosphate synthase (EC 2.5.1.83), all-trans-nonaprenyl diphosphate synthase (EC 2.5.1.84), all-trans-nonaprenyl diphosphate synthase (EC 2.5.1.85), trans,polycis-decaprenyl diphosphate synthase (EC 2.5.1.86), ditrans,polycis-polyprenyl diphosphate synthase (EC 2.5.1.87), trans,polycis-polyprenyl diphosphate synthase (EC 2.5.1.88), tritrans,polycis-undecaprenyl-diphosphate synthase (EC 2.5.1.89), octaprenyl diphosphate synthase (EC 2.5.1.90), decaprenyl diphosphate synthase (EC 2.5.1.91), (2Z,6Z)-farnesyl-diphosphate synthase (EC 2.5.1.92), and 4-hydroxybenzoate geranyltransferase (EC 2.5.1.93).

A preferred example of a prenyltransferase is a dimethylallyltransferase.

In one aspect of the present invention, the prenyltransferase is selected from isoprenylpyrophosphate synthases catalysing a reaction of a first substrate with a second substrate, said first substrate being selected from isopentenyl pyrophosphate (IPP), dimethyl allyl pyrophosphate (DMAPP), geranyl pyrophosphate (GPP), farnesyl pyrophosphate (FPP), and geranylgeranyl pyrophosphate (GGPP), and said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate.

Isoprenylpyrophosphate synthases can catalyse the chain elongation of allylic isoprenyl pyrophophate substrates via consecutive condensation reactions with isopentenyl pyrophosphate or/and other isoprenoid moieties to generate linear polymers with defined chain length (for review, see Liang PH, Ko TP Wang AH, Structure, mechanism and function of prenyltransferases, Eur J Biochem 2002;269(14):3339-54).

The isoprenylpyrophosphate synthase of the present invention can be selected from *cis*-isoprenylpyrophosphate synthases, and *trans-*isoprenylpyrophosphate synthases.

The isoprenylpyrophosphate synthase of the present invention is in particular selected from dimethylallyltranstransferase (EC 2.5.1.1), (2E, 6E)-farnesyl diphosphate synthase (EC 2.5.1.10), rubber cis-polyprenylcistransferase (EC 2.5.1.20), squalene synthase (EC 2.5.1.21), dimethylallylcistransferase (EC 2.5.1.28), farnesyltranstransferase (EC 2.5.1.29), heptaprenyl diphosphate synthase (EC 2.5.1.30), ditrans,polycis-Undecaprenyl diphosphate synthase (EC 2.5.1.31), phytoene synthase (EC 2.5.1.32), geranylgeranylglycerolphosphate-geranylgeranyltransferase (EC 2.5.1.42), chrysanthemyl diphosphate synthase (EC 2.5.1.67), (2Z,6E)-farnesyl diphosphate synthase (EC 2.5.1.68), lavandulyl diphosphate synthase (EC 2.5.1.69), geranylfarnesyl diphosphate synthase (EC 2.5.1.81), hexaprenyl diphosphate synthase (EC 2.5.1.82), hexaprenyl diphosphate synthase (EC 2.5.1.83), all-trans-nonaprenyl diphosphate synthase (EC 2.5.1.84), all-trans-nonaprenyl diphosphate synthase (EC 2.5.1.85), trans,polycis-decaprenyl diphosphate synthase (EC 2.5.1.86), ditrans,polycis-polyprenyl diphosphate synthase (EC 2.5.1.87), trans,polycis-polyprenyl diphosphate synthase (EC 2.5.1.88), tritrans,polycis-undecaprenyl-diphosphate synthase (EC 2.5.1.89), octaprenyl diphosphate synthase (EC 2.5.1.90), decaprenyl diphosphate synthase (EC 2.5.1.91), (2Z,6Z)-farnesyl-diphosphate synthase (EC 2.5.1.92).

In another aspect of the present invention, the prenyltransferase is selected from protein prenyltransferases catalysing a reaction of a first substrate with a second substrate, said first substrate being selected from proteins, and said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate. A preferred substrate of protein prenyltransferases is selected from farnesyl pyrophosphate and geranylgeranyl pyrophosphate.

Protein prenyltransferases can catalyse the attachment of either a farnesyl group or a geranylgeranyl group via a thioether linkage (-C-S-C-) to a cysteine at or near the carboxyl terminus of the protein. There are three different protein prenyltransferases: farnesyltransferase (FT) and geranylgeranyltransferase 1 (GGT1) share the same motif (the CaaX box) around the cysteine in their substrates, and are thus called CaaX prenyltransferases, whereas geranylgeranyltransferase 2 (GGT2, also called Rab geranylgeranyltransferase) recognizes a different motif and is thus called a non-CaaX prenyltransferase. Each protein consists of two subunits, and the subunit of FT and GGT1 is encoded by the same gene, FNTA (for review of protein prenyltransferases, see Maurer-Stroh et al., Genome Biology 2003, 4:212).

In the present invention, the protein prenyltransferase can be selected from protein prenyltransferases disclosed in Maurer-Stroh et al. (Genome Biology 2003, 4:212), the disclosure of which is included herein by reference. In the present invention, the protein prenyltransferase is preferably selected from protein farnesyltransferase (FT, EC 2.5.1.58), protein geranylgeranyltransferase 1 (GGT1, also termed protein geranylgeranyltransferase type I, GGTase I, EC 2.5.1.59), and geranylgeranyltransferase 2 (GGT2, also termed geranylgeranyltransferase type II, RabGGTase, EC 2.5.1.60). If the selected protein prenyltransferase is farnesyltransferase (FT), the second substrate is preferably farnesyl pyrophosphate. If the selected protein prenyltransferase is geranylgeranyltransferase 1 (GGT1) or geranylgeranyltransferase 2 (GGT2), the second substrate is preferably geranylgeranyl pyrophosphate.

The protein prenyltransferase preferably catalyses coupling at a cysteine residue which may be located at or near the carboxyl terminus of the protein. In this context "near the carboxyl terminus" in particular refers to a cysteine located at a position located 1, 2, 3, 4, 5, 6, 7, 8, 9, 10, at the maximum 15, at the maximum 20, or at the maximum 30 amino acid residues distant from the carboxy terminus.

In particular, the protein farnesyltransferase (FT) and geranylgeranyltransferase 1 (GGT1) can catalyse coupling at a cysteine residue located in the motif Ca₁a₂X, wherein "C" is cystein, "a₁" and "a₂" are preferably aliphatic amino acids residues, and "X" is C, S, Q, A, M, T, H, V, N, F, G or I for protein farnesyltransferase, or "X" is L, F, I, V or M for protein geranylgeranytransferase GGT1. The Ca₁a₂X motif may be located at or near the carboxyl terminus of the protein, as described herein.

In particular, geranylgeranyltransferase GGT2 can catalyse coupling at a cysteine residue located in a motif selected from CC, CXC, CCX, CCXX, CCXXX and CXXX, wherein C is cysteine and X can be independently selected from any amino acids present in proteins. This motif may be located at or near the carboxyl terminus of the protein, as described herein. Any cysteine residue in the motif may be used for coupling.

Any protein providing cysteine residue which may be located at or near the carboxyl terminus of the protein can be used as a substrate for the protein prenyltransferase. In particular, a protein providing a Ca₁a₂X motif or/and a motif selected from CC, CXC, CCX, CCXX, CCXXX and CXXX, as described herein, can be prenylated.

In yet another aspect of the present invention, the prenyltransferase is selected from aromatic prenyltransferases catalysing a reaction of a first substrate with a second substrate, said first substrate being selected from compounds comprising an aromatic group, and said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate.

The aromatic prenyl transferase is preferably selected from 4-dimethylallyltryptophan synthase (EC 2.5.1.34), aspulvinone dimethylallyltransferase (EC 2.5.1.35), trihydroxypterocarpan dimethylallyltransferase (EC 2.5.1.36), 4-hydroxybenzoate polyprenyltransferase (EC 2.5.1.39), naringenin-8-dimethylallyltransferase (EC 2.5.1.70), leachianon-G2"-dimethylallyltransferase (EC 2.5.1.71), 1,4-dihydroxy-2-naphtoat polyprenyltransferase (EC 2.5.1.74), 7-dimethylallyltryptophan synthase (EC 2.5.1.80), 4-hydroxybenzoate geranyltransferase (EC.2.5.1.93).

A preferred aromatic prenyltransferase can be obtained from *Aspergillus fumigatus.* More preferably, the aromatic prenyltransferase is selected from 7-dimethylallyltryptophan synthase (EC 2.5.1.80) and 4-dimethylallyltryptophan synthase (EC 2.5.1.34). An example of a 4-dimethylallyltryptophan synthase is the FgaPT2 prenyltransferase. The FgaPT2 prenyltransferase may comprise amino acid position 3 to 461 of SEQ ID NO:2 or a partial sequence thereof having 4-dimethylallyltryptophan synthase activity. The partial sequence may have a length of at least 200 amino acid residues, at least 300 amino acid residues, at least 400 amino acid residues, or at least 450 amino acid residues.

Known aromatic prenyltransferases show a low substrate specificity and are thus able to accept a rather broad diversity of substrates. See, for example, references [7] and [13], the disclosure of which is included herein by reference.

Compounds comprising an aromatic group, as used herein, include indole and derivatives thereof. Preferred indole derivatives include tryptophan and derivatives thereof. Coupling of the allylic isoprenyl pyrophosphate substrate at the indole may take place at position 4 or 7 of the indole moiety.

In the present invention, tryptophan includes D-tryptophan and L-tryptophan, and mixtures thereof.

A preferred substrate of 7-dimethylallyltryptophan synthase and 4-dimethylallyltryptophan synthase is L-tryptophan.

In the present invention, "indole derivative" refers to a compound wherein the indole moiety comprises at least one substituent located at a position independently selected from position 1, 2, 3, 4, 5, 6, 7. It is preferred that the at least one substituent is located at a position independently selected from position 1, 2, 3, 5, 6, 7 or at a position independently selected from position 1, 2, 3, 4, 5, 6. Examples of indole derivatives are tryptophan, indole-3-propionic acid and L-β-homotryptophan. Positions 1 to 7 of the indole molecule are defined according to the IUPAC nomenclature.

In the present invention, in an indole derivative may comprise at position 1 a C₁₋₆ alkyl group, in particular methyl.

In the present invention, in an indole derivative may comprise at position 5 a substituent selected from halogen, hydroxy, and C₁₋₆alkyl. In particular, the C₁₋₆ alkyl at position 5 is methyl.

In the present invention, in an indole derivative may comprise at position 6 a substituent selected from halogen and C₁₋₆alkyl. In particular, the C₁₋₆ alkyl at position 6 is methyl.

In the present invention, in an indole derivative may comprise at position 7 a C₁₋₆ alkyl group. In particular, the C₁₋₆alkyl at position 7 is methyl.

The halogen can be selected from fluoro, chloro, bromo, and iodo. A preferred halogen is fluoro. Another preferred halogen is chloro. Yet another preferred halogen is bromo. A further preferred halogen is iodo.

Tryptophan can be considered as alanine substituted by indole. In the present invention, "tryptophan derivative" refers to a compound wherein the tryptophan moiety is substituted at the indole moiety, as described herein, or/and which comprises at least one substituent located at the alanyl moiety, as, for example, described in Kremer et al., Appl Microbiol Biotechnol. 2008 Jul;79(6):951-61.

Indole derivatives including tryptophan and derivatives thereof suitable as substrates of 4-dimethylallyltryptophan synthase are described by Steffan et al. (Chemoenzymatic synthesis of prenylated indole derivatives by using a 4-dimethylallyltryptophan synthase from Aspergillus fumigatus, ChemBioChem 2007 Jul 23;8(11):1298-307), the disclosure of which is included herein by reference.

Indole derivatives including tryptophan and derivatives thereof suitable as substrates of 7-dimethylallyltryptophan synthase are described by Kremer and Li (Potential of a 7-dimethylallyltryptophan synthase as a tool for production of prenylated indole derivatives, Appl Microbiol Biotechnol. 2008 Jul;79(6):951-61), the disclosure of which is included herein by reference.

Compounds comprising an aromatic group, as used herein, include flavonoids. For example, the flavonoids apigenin and liquiritigenin can be prenylated as described herein. Another example is the flavonoid narringenin. Narringenin-8-dimethylallyl transferase (EC 2.5.1.70) isolated from *Sophora flavescens* (Fabaceae) catalyses the transfer of DMAPP to the flavonoid narringenin. Another aromatic substrate of aromatic prenyl transferases is 4-hydroxybenzoate. The enzyme 4-hydroxybenzoate geranyltransferase (EC 2.5.1.93) catalyses the transfer of GPP to 4-hydroxybenzoate. Yet another example is 4-hydroxyphenylpyruvic acid. The enzyme CloQ from *Streptomyces* catalyses the transfer of DMAPP to 4-hydroxyphenylpyruvic acid. The aromatic prenyltransferase and the corresponding substrates can be selected from aromatic prenyltransferases disclosed by Heide et al. (Current Opinion in Chemical Biology 2009, 13:171-179), the disclosure of which is included herein by reference.

Furthermore, compounds comprising an aromatic groups, which can be prenylated according to the present invention, include oligopeptides having from 2 to 20, from 2 to 15 or from 2 to 10 amino acid residues and comprising at least one tryptophan residue. Preferred is an oligopeptide selected from dipeptides comprising at least one tryptophan residue, tripeptides comprising at least one tryptophan residue and tetrapeptides comprising at least one tryptophan residue. The oligopeptide may be a cyclic oligopeptide comprising at least one tryptophan residue. In particular, the oligopeptide is cyclic dipeptide comprising at least one tryptophan residue. A more preferred dipeptide is *cyclo*-L-Trp-L-Pro.

Preferably, an IPP or an DMAPP residue is coupled at the at least one tryptophan residue of an oligopeptide as described herein.

By the prenylation as described herein, tryprostatins can be obtained by prenylation of *cyclo*-L-Trp-L-Pro. Examples of tryprostatins include tryprostatin A and tryprostatin B.

The prenylation of the present invention is preferably performed at room temperature. The temperature can be a temperature selected from the ranges of about 16°C to about 24°C, about 17°C to about 23°C, about 18°C to about 22°C, and about 19°C to about 21°C. Preferred is a temperature of about 20°C.

The recombinant prenyltransferase to be displayed may also be termed "passenger", "passenger polypeptide" or "passenger protein".

Step (a) of the methods of the present invention refers to the provision of a host cell. The host cell used in the method of the present invention is preferably a bacterium, more preferably a gram-negative bacterium, particularly an enterobacterium such as *E. coli*.

According to the present invention, a host cell, particularly a host bacterium is provided which is transformed with a nucleic acid fusion operatively linked with an expression control sequence, i.e. a promoter, and optionally further sequences required for gene expression in the respective host cell. The skilled person knows suitable promoters and expression control sequences. The promoter or/and the expression control sequence may be homologous or heterologous to the host cell. The promoter or/and the expression control sequence are preferably homologous to the host cell.

Preferably, the nucleic acid fusion is located on a recombinant vector, e.g. a plasmid vector.

The nucleic acid fusion comprises (i) a portion encoding a signal peptide, preferably a portion coding for a gram-negative signal peptide allowing for transport into the periplasm through the inner cell membrane. The signal peptide may be a signal peptide homologous to the host cell. The signal peptide may also be a signal peptide heterologous to the host cell.

Further, the nucleic acid fusion comprises (ii) a portion encoding the recombinant prenyltransferase to be displayed.

Further, the nucleic acid fusion optionally comprises (iii) a portion encoding a protease recognition site, which may be a recognition site for an intrinsic protease, i.e. a protease naturally occurring in the host cell, or an externally added protease. For example, the externally added protease may be an IgA protease (cf. EP-A-0 254 090), thrombin or factor X. The intrinsic protease may be e.g. selected from OmpT, OmpK or protease X.

Furthermore, the nucleic acid fusion comprises (iv) a portion encoding a transmembrane linker which is required for the presentation of the passenger polypeptide (ii) on the outer surface of the outer membrane of the host cell. A transmembrane linker domain may be used which is homologous with regard to the autotransporter, i.e. the transmembrane linker domain is encoded by a nucleic acid portion directly 5' to the autotransporter domain. Also a transmembrane linker domain may be used which is heterologous with regard to the autotransporter. The length of the transmembrane linker is preferably 30-160 amino acids.

Further, the nucleic acid fusion comprises (v) a transporter domain of an autotransporter. In the context of the present invention, autodisplay may be the recombinant surface display of proteins or polypeptides by means of an autotransporter in any Gram-negative bacterium. The transporter domain of the autotransporter according to the invention can be any transporter domain of an autotransporter and is preferably capable of forming a β-barrel structure. A detailed description of the β-barrel structure and preferred examples of β-barrel autotransporters are disclosed in WO97/35022 incorporated herein by reference. Henderson et al. (2004) describes autotransporter proteins which comprise suitable autotransporter domains (for summary, see Table 1 of Henderson et al., 2004). The disclosure of Henderson et al. (2004) is included herein by reference. For example, the transporter domain of the autotransporter may be selected from Ssp (P09489, *S*. *marcescens*), Ssp-h1 (BAA33455, *S*. *marcescens*), Ssp-h2 (BAA11383, *S*. *marcescens*), PspA (BAA36466, *P*. *fluorescens*), PspB (BAA36467, *P*. *fluorescens*), Ssa1 (AAA80490, *P*. *haemolytica*), SphB1 (CAC44081, *B. pertussis*), AspA/NalP (AAN71715, *N. meningitidis*), VacA (Q48247, *H. pylori*), AIDA-I (Q03155, *E*. *coli*), IcsA (AAA26547, *S*. *flexneri*), MisL (AAD16954, S. *enterica*), TibA (AAD41751, *E*. *coli*), Ag43 (P39180, *E*. *coli*), ShdA (AAD25110, *S*. *enterica),* AutA (CAB89117, *N*. *meningitidis*), Tsh (154632, *E. coli*), SepA (CAC05786, S. flexneri), EspC (AAC44731, *E*. *coli*), EspP (CAA66144, *E*. *coli*), Pet (AAC26634, *E*. *coli*), Pic (AAD23953, *E*. *coli*), SigA (AAF67320, S. flexneri), Sat (AAG30168, *E. coli*), Vat (AAO21903, *E*. *coli*), EpeA (AAL18821, *E*. *coli*), EatA (AA017297, *E*. *coli*), EspI (CAC39286, *E. coli*), EaaA (AAF63237, *E*. *coli*), EaaC (AAF63038, *E*. *coli*), Pertactin (P14283, *B. pertussis*), BrkA (AAA51646, *B. pertussis*), Tef (AAQ82668, B. *pertussis*), Vag8 (AAC31247, *B. pertussis*), PmpD (084818, *C. trachomatis*), Pmp20 (Q9Z812, C. *pneumoniae*), Pmp21 (Q9Z6U5, *C*. *pneumoniae*), IgA1 protease (NP_283693, *N. meningitidis*), App (CAC14670, *N*. *meningitidis*), IgA1 protease (P45386, *H*. *influenzae*), Hap (P45387, *H*. *influenzae*), rOmpA (P15921, *R. rickettsii*), rOmpB (Q53047, *R. rickettsii*), ApeE (AAC38796, *S*. *enterica*), EstA (AAB61674, *P. aeruginosa*), Lip-1 (P40601, *X*. *luminescens*), McaP (AAP97134, *M*. *catarrhalis*), BabA (AAC38081, *H. pylori*), SabA (AAD06240, *H. pylori*), AlpA (CAB05386, *H. pylori*), Aae (AAP21063, *A*. *actinomycetemcomitans*), NanB (AAG35309, *P*. *haemolytica*), and variants of these autotransporters having at least 90 % identity thereto. Given in brackets for each of the exemplary autotransporter proteins are examples of suitable genbank accession numbers and species from which the autotransporter may be obtained. Preferably the transporter domain of the autotransporter is the *E*. *coli* AIDA-I protein or a variant thereof having at least 90 % identity thereto. AIDA-I is e.g. described by Niewert U., Frey A., Voss T., Le Bouguen C., Baljer G., Franke S., Schmidt MA. The AIDA Autotransporter System is Associated with F18 and Stx2e in Escherichia coli Isolates from Pigs Diagnosed with Edema Disease and Postweaning Diarrhea. Clin. Diagn. Lab. Immunol. 2001 Jan, 8(1):143-149;9.

Variants of the above indicated autotransporter sequences can e.g. be obtained by altering the amino acid sequence in the loop structures of the β-barrel not participating in the transmembrane portions. Optionally, the nucleic acid portions coding for the surface loops can be deleted completely. Also within the amphipathic β-sheet conserved amino exchanges, i.e. the exchange of an hydrophilic by another hydrophilic amino acid or/and the exchange of a hydrophobic by another hydrophobic amino acid may take place. Preferably, a variant has a sequence identity of at least 90%, at least 95% or at least 98% on the amino acid level to the respective native sequence of the autotransporter domain, in particular in the range of the β-sheets.

Step (b) of the methods of the present invention refers to culturing the host cell under conditions wherein the nucleic acid fusion is expressed and the expression product comprising the recombinant prenyltransferase is displayed on the surface of the host cell. The person skilled in the art knows suitable culture conditions. The method according to the invention allows for an efficient expression of passenger proteins on the surface of host cells, particularly *E. coli* or other gram-negative bacterial cells up to 100 000 or more molecules per cell by using a liquid medium of the following composition: 5 g/l to 20 g/l, preferably about 10 g/l trypton, 2 g/l to 10 g/l, preferably about 5 g/l yeast extract, 5 g/l to 20 g/l, in particular about 10 g/l NaCl and the remaning part water. The medium should possibly contain as little as possible divalent cations, thus preferably Aqua bidest or highly purified water, e.g. Millipore water is used. The liquid medium may contain in addition preferably EDTA in a concentration of 2 µM to 20 µM, in particular 10 µM. Moreover, it contains preferably reducing reagents, such as 2-mercapto ethanol or dithiotreitol or dithioerythritol in a preferred concentration of 2 mM to 20 mM. The reducing reagents favour a non-folded structure of the polypeptide during transport. The liquid medium can further contain additional C-sources, preferably glucose, e.g. in an amount of up to 10 g/l, in order to favour secretion i.e. transfer of the passenger to the surrounding medium. For surface display preferably no additional C-source is added. Preferred culture conditions for Gram-negative cells, such as *E*. *coli*, are described in the Examples.

If the host cell is a gram-negative bacterium, the polypeptide synthesized in the cytoplasma can be translocated from the cytoplasm into the periplasmic space by crossing the inner membrane. This can be effected by the signal peptide.

The components (i) to (v) in the nucleic acid fusion of the present invention are preferably oriented from 5' to 3'. In the expression product obtained in step (b), the amino acid sequences encoded by nucleic acid sequences (i) to (v) are preferably arranged N terminal to C terminal.

The method of the present invention may comprise preparing a membrane preparation from the cell obtained in step (b). The membrane preparation may comprise membrane particles. The membrane particles may be membrane vesicles. Preferred membrane particles are outer membrane particles. In particular the method of the present invention may comprise preparing outer membrane particles of cells displaying a recombinant polypeptide on the surface, e.g. of Gram-negative bacterial cells. The person skilled in the art knows suitable conditions (e.g. Hantke, 1981, Schultheiss et al., 2002). Typical conditions for preparing membrane particles are employed in the examples of the present invention. Outer membrane particles from a host cell as described herein may be performed by a method comprising the steps:
(a) treating the host cell with a hydrolase (such as lysozyme) and optionally with a DNAse. This enzymatic treatment may be performed at room temperature. The hydrolase hydrolyses the cell wall within the periplasmic space. The cell wall comprises peptidoglycans to be hydrolyzed.
(b) optionally solubilizing the preparation of (a) with a tenside, such as Triton X-100, or/and with sarcosine, followed by optional centrifugation of cell debris. The thus obtained preparation of outer membrane particles may be centrifuged, washed and resuspended.

The diameter of the membrane particles may be in the range of 1 nm to 1000 nm, in the range of 50 nm to 500 nm, in the range of 75 to 200 nm, or in the range of 90 to 120 nm. At least 80%, at least 90%, at least 95 %, or at least 98% of the membrane particles may have a diameter in a range selected from the ranges described herein.

In a host cell being a Gram-negative bacterium, such as *E. coli*, after translocation, the recombinant passenger remains attached to the surface of the outer membrane by the β-barrel, which is serving as an anchor within the outer membrane. Due to the controlled integration of the β-barrel within the outer membrane, the C terminal part of the β-barrel is directed to the inner side of the outer membrane, whereas the N-terminal part of the linker, to which the recombinant passenger protein is covalently bound, is directed to the outer surface of the outer membrane, i.e. the environment. The recombinant passenger protein has an oriented location after transport, namely it is directed to the cellular surface. The recombinant passenger protein has the identical orientation as the lipopolysaccharide (LPS) layer which may be present in the outer membrane.

Membrane particles of the present invention prepared from the host cell of the present invention comprise the recombinant prenyltransferase at the surface directed to the environment. In contrast to the inner membrane which is a unit membrane, the outer membrane of Gram-negative bacteria, in particular *E. coli*, is asymmetric. The outer membrane may comprise an inner layer comprising phospholipids and an outer layer comprising LPS. LPS is hydrophilic and may contain several negative charges. By using outer membrane particles with anchored passenger proteins by a β-barrel for the coating of carriers, the outer side of the outer membrane, in particular the LPS side will be directed to the surface distal to the carrier. As a consequence the recombinant protein or a domain thereof, which are integrated in the outer membrane by autodisplay, will be directed to the surface distal to the carrier as well. The core part of the membrane particles may stabilize the interaction of the outer membrane layer obtained by applying outer membrane particles to the carrier by hydrophobic interactions and may contain lipoproteins or peptidoglycans.

In a preferred embodiment, the prenyltransferase comprises a sequence selected from
(a) amino acid position 3 to 461 of SEQ ID NO:2 and
(b) sequences having at least 70%, at least 80%, at least 90%, at least 95%, or at least 98% identity to the sequence of (a).

The portion (ii) of the nucleic acid fusion of the present invention may encode a prenyltransferase, as described herein. The portion (ii) of the nucleic acid fusion of the present invention may comprise a nucleic acid sequence selected from
(a) nucleic acid sequences encoding the sequence of amino acid position 3 to 461 of SEQ ID NO:2,
(b) nucleic acid sequences encoding amino acid sequences having at least 70%, at least 80%, at least 90%, at least 95%, or at least 98% identity to the amino acid sequence of SEQ ID NO:2,
(c) nucleotide position 7 to 1383 of SEQ ID NO:1, and
(d) sequences having at least 70%, at least 80%, at least 90%, at least 95%, or at least 98% identity to the sequence of (c).

In particular, nucleic acid sequences of (a), (b) and (d) include sequences within the scope of the degeneracy of the genetic code.

The skilled person knows suitable methods to determine the degree of identity of nucleic acid sequences and amino acid sequences. Known algorithms, such as BLAST (for nucleic acids) or PBLAST (for amino acid sequences) may be used. A nucleic acid or polypeptide comprising sequences having at least 70%, at least 80%, at least 90%, at least 95%, or at least 98% identity to a given sequence includes fragments of the given nucleic acid or polypeptide.

The prenyltransferase of the present invention to be displayed on the surface of the cell may be a monomeric or dimeric polypeptide. The dimeric recombinant polypeptide may be a homodimer, i.e. a polypeptide consisting of two identical subunits. On the other hand, the dimeric recombinant polypeptide may be a heterodimer, i.e. a polypeptide consisting of two different subunits. The nucleic acid fusion of the present invention may encode two polypeptide subunits as a polypeptide fusion which when presented on the cell surface forms a functional dimeric polypeptide.

Preferably, the prenyltransferase can form an active dimer on the surface of the host cell. Homodimers may be formed by a spontaneous association of two identical polypeptide subunits displayed on the host cell membrane. Heterodimers may be formed by a spontaneous association of two different polypeptide subunits displayed on the host cell membrane.

Examples of prenyltransferases which can form active dimers include protein prenyltransferases, as described herein, and aromatic prenyl transferases as disclosed herein, such as FgaPT2.

On the other hand, a dimeric recombinant polypeptide may be formed by an association of one polypeptide subunit displayed on the host cell membrane, as described herein, and one soluble polypeptide subunit added to the host cell membrane. The added subunit may be identical to the displayed subunit or be different therefrom.

Yet another aspect of the present invention is a host cell displaying the recombinant polypeptide of the present invention on the surface. The host cell may be any host cell as described herein. In particular, the host cell displays a recombinant polypeptide comprising a prenyltransferase on the surface of the host cell, wherein the recombinant polypeptide comprises
(I) a portion comprising the recombinant prenyltransferase to be displayed,
(II) optionally a portion comprising a protease recognition site,
(III) a portion comprising a transmembrane linker, and
(IV) a portion comprising the transporter domain of an autotransporter.

The portions (I) to (IV) of the recombinant polypeptide displayed by the host cell of the present invention are encoded in particular by the components (ii), (iii), (iv) and (v) of the nucleic fusion, as described herein.

Yet another aspect of the present invention is a membrane preparation comprising a recombinant polypeptide. The membrane preparation of the present invention may comprise membrane particles, as described herein. The membrane preparation may be obtained from a host cell as described herein. The recombinant prenyltransferase may be any recombinant prenyltransferase as described herein.

Yet another aspect of the present invention is the use of a membrane preparation comprising a recombinant polypeptide in the manufacture of a carrier comprising a recombinant polypeptide.

The membrane preparation of the present invention may be employed for coating a carrier. The carrier may comprise a membrane preparation of the present invention, as described herein.

The carrier may comprise a hydrophobic surface. The hydrophobic surface may have a contact angle of more than 90°. A increasing surface angle of more than 30° indicates a gradually increasing hydrophobicity of a surface. In the present context, a hydrophobic surface may have a contact angle of at least 40°. The surface preferably has a hydrophobicity described by a contact angle of at least 40°, at least 50°, at least 60°, at least 65°, at least 70°. Contact angles are preferably determined by the sessile drop method. The sessile drop method is a standard method for determining contact angles.

Measurements may be performed with a contact angle goniometer. Preferred contact angles of the hydrophobic surface are in a range of 40° to 100°, 50° to 90°, or 60° to 80°.

The surface of the carrier may be a metal surface. A suitable metal surface has a contact angle e.g. in the range of 50° to 80°. A suitable metal may be selected from gold, silver, titanium, aluminium and alloys such as brass. A preferred surface is a gold surface. The gold surface may be employed as it is. An untreated gold surface has a hydrophobicity suitable for the carrier as described herein. A treatment of the gold surface with thiolated hydrocarbons or hydrocarbons with functional groups such as carboxylic acids or hydroxyl groups is not required.

Another preferred surface of the carrier comprises a polymer, for instance a surface usually employed in disposable materials for use in biochemical or/and medical science. The polymer may be an artificial polymer. Examples of artificial polymers include a polymer selected from polystyrenes, polypropylenes, and polycarbonates. The polystyrene may be produced from [2,2]paracyclophane monomers. Polystyrene surfaces may be treated with oxygene plasma introducing OH or/and methylene groups in order to modify the hydrophobicity. Examples of such modified surfaces include Maxi-sorp, Medi-sorp, Multi-sorp, and Poly-sorp surfaces. Another suitable polystyrene surface is Parylene N produced from [2,2]paracyclbphane monomers. Yet another suitable surface is Parylene A [Poly(monoamino-p-xylene)]. Especially suitable are surfaces comprising a polymer having a hydrophobicity described by a contact angle of at least 50°. Suitable surfaces are selected from polystyrene, Parylene A, Parylene N, Maxi-sorp, Medi-sorp, Multi-sorp, and Poly-sorp. Preferred surfaces are selected from polystyrene, Parylene A, Parylene N, Maxi-sorp, Medi-sorp, and Poly-sorp.

The surface may comprise a natural polymer. Suitable natural polymers include polybutyrate and cellulose and derivatives thereof. A further surface is provided by latex particles, in particular latex beads.

Yet another surface is provided by C18-modified particles, in particular C18-modified monolithic silica particles. C18 refers to an alkyl group comprising 18 carbon atoms. C18-modified particles are known in the art.

Yet another suitable surface is a glass surface.

The surface may be modified is order to adjust the hydrophobicity. Modification may be performed by chemical treatment (i.e. by oxygen plasma), physical treatment (e.g. by laser irradiation or/and radioactive irradiation), or by mechanical treatment.

The host cell as described herein or/and the membrane preparation as described herein can be used in a prenylation reaction, as described herein.

The method according to the invention and the host cells according to the invention can be used for a variety of different applications, e.g. as whole cell biofactories or membrane preparation biofactories for chemical synthesis procedures, e.g. for the synthesis of organic substances selected from enzyme substrates, drugs, hormones, starting materials and intermediates for syntheses procedures and chiral substances (cf. Roberts, Chemistry and Biology 6 (1999), R269-R272).

Furthermore, the cell or the membrane preparation of the invention may be used for a directed evolution procedure, e.g. for the development of new biocatalysts for the application in organic syntheses.

This is achieved in a particular embodiment by varying the amino acid sequence of the prenyltransferase, as described herein, via site-specific or random mutagenesis and by testing variant carrying cells or membrane preparations or libraries containing variant carrying cells or membrane preparations thereof using a certain chemical reaction with the help of suitable screening methods, in particular high throughput screening (HTS) methods for the conversion of a certain substrate.

In yet another preferred embodiments libraries of variants of a prenyltransferase, as described herein, are examined in view of the role of defined amino acids during certain functions, in particular catalytic functions.

In general, these particular embodiments concern the production of variants of prenyltransferases and the production of libraries with variants of prenyltransferases which are screened in view of a certain characteristic, i.e. one or optionally several variants fulfilling this desired characteristic perfectly are selected. By selecting the variant the cell is selected, too, and carries the nucleic acid coding the variant. Thus, at the same time both the amino acid sequence and the structural information of the variant can be determined via the nucleic acid sequence. The characteristics in question are particularly enzyme inhibiting, catalytical, toxin degrading, synthesizing, therapeutical etc. characteristics.

Moreover, the host cell or the membrane preparation may be used as an assay system for a screening procedure, e.g. for identifying modulators (activators or inhibitors) of prenyltransferases, as described herein, which may be used as potential therapeutic agents. The screening procedure may also be used to identify variants of displayed polypeptides having predetermined desired characteristics. For this purpose, libraries of modulators and/or libraries of displayed prenyltransferases may be used. Further, the host cells or membrane preparations derived therefrom may be used as a system for toxicity monitoring and/or degrading toxic substances in the environment, in the laboratory or in biological, e.g. human, animal, or non-biological systems.

An essential advantage of applying the host cells and membranes according to the invention is enabling correct folding and biological activity of proteins or protein complexes, e.g. of the prenyltransferases, as described herein, which require a membrane environment. Thus, a reconstitution as previously considered to be necessary is no longer required. Thereby the production steps of a functional biocatalytic system are simplified and an increased stability of the system *per se* is obtained.

With the help of the host cell and/or membrane preparation according to the invention it can be found out in an early step of drug discovery, the so-called lead identification, whether the new drug lead structure to be tested could possibly have side-effects or lead to the so-called drug-drug interaction.

Yet another aspect of the present invention is a method for producing a microorganism displaying a recombinant prenyltransferase on its surface comprising introducing a nucleic acid into said microorganism, said nucleic acid comprising:
(i) a portion encoding a signal peptide,
(ii) a portion encoding the recombinant prenyltransferase to be displayed,
(iii) optionally a portion encoding a protease recognition site,
(iv) a portion encoding a transmembrane linker, and
(v) a portion encoding the transporter domain of an autotransporter, wherein the nucleic acid is operatively linked with an expression control sequence. The microorganism may be a microorganism selected from bacteria, particularly gram-negative bacteria, more particularly enterobacteria. A preferred example is *E*. *coli*.

Yet another aspect of the present invention is a method for prenylation of a first substrate by a prenyltransferase, said method comprising the steps
(a) providing a host cell comprising the prenyl transferase located on its surface, or/and a membrane preparation of the host cell, and
(b) contacting the host cell or/and the membrane preparation thereof with a first substrate and a second substrate, under conditions wherein the prenyltransferase catalyses the reaction of the first substrate and the second substrate, and wherein the second substrate is coupled to the first substrate.

In the aspects of the present invention, in particular in the method for prenylation of a first substrate by a prenyltransferase, a preferred first substrate is IPP.

In the aspects of the present invention, in particular in the method for prenylation of a first substrate by a prenyltransferase, the second substrate is preferably selected from allylic isoprenyl pyrophosphates, as described herein.

In the aspects of the present invention, in particular in the method for prenylation of a first substrate by a prenyltransferase, the first and second substrate can be identical or different. For example, the squalene synthase (EC 2.5.1.21) requires FPP as a first and second substrate. In this example, the first and second substrates are identical. In another example, dimethylallyl-*trans*-transferase (EC 2.5.1.1) or dimethylallyl-*cis*-transferase (EC 2.5.1.28) require IPP and DMAPP as a first and second substrate. In this example, the first and second substrate are different. Other examples of identical and different first and second substrates are disclosed herein.

In the aspects of the present invention, in particular in the method for prenylation of a first substrate by a prenyltransferase, the prenyltransferase may be any known prenyltransferase. The skilled person knows typical substrates of prenyltransferases, which can be used as first and second substrates.

In the aspects of the present invention, in particular in the method for prenylation of a first substrate by a prenyltransferase, the prenyltransferase may be selected from the prenyltransferases as described herein. The skilled person knows suitable substrates of the selected prenyltransferase. In the aspects of the present invention, in particular in the method for prenylation of a first substrate by a prenyltransferase, combinations of a prenyltransferase and first and second substrates may be selected from the following combinations: dimethylallyltranstransferase (EC 2.5.1.1) combined with substrates DMAPP and IPP; (2E, 6E)-farnesyl diphosphate synthase (EC 2.5.1.10) combined with substrates GPP and IPP; rubber cis-polyprenylcistransferase (EC 2.5.1.20) combined with substrates poly-cis-polyprenyl diphosphate and IPP (rubber particles can be used as prenyl acceptors); squalene synthase (EC 2.5.1.21) combined with substrates FPP and FPP; dimethylallylcistransferase (EC 2.5.1.28) combined with substrates DMAPP and IPP; farnesyltranstransferase (EC 2.5.1.29) combined with substrates transtrans FPP and IPP; heptaprenyl diphosphate synthase (EC 2.5.1.30) combined with substrates FPP and 4 IPP; ditrans,polycis-Undecaprenyl diphosphate synthase (EC 2.5.1.31) combined with substrates FPP and 8 IPP; phytoene synthase (EC 2.5.1.32) combined with substrates GGPP and GGPP; 4-dimethylallyltryptophan synthase (EC 2.5.1.34) combined with substrates DMAPP and L-tryptophan; aspulvinone dimethylallyltransferase (EC 2.5.1.35) combined with substrates 2 DMAPP and aspulvinone E; trihydroxypterocarpan dimethylallyltransferase (EC 2.5.1.36) combined with substrates DMAPP and (6aS,11a,S)-3,6a,9-trihydroxypterocarpan; 4-hydroxybenzoate polyprenyltransferase (EC 2.5.1.39) combined with substrates polyprenyldiphosphate and 4-hydroxybenzoate; phosphoglycerol geranylgeranyltransferase (EC 2.5.1.41) combined with substrates GGPP and sn-glycerol-1-phosphate; geranylgeranylglycerol-phosphate-geranylgeranyltransferase (EC 2.5.1.42) combined with substrates GGPP and sn-3-O-(geranylgeranyl)glycerol 1-phosphate; protein farnesyltransferase (EC 2.5.1.58) combined with substrates FPP and a protein comprising a cystein, as described herein; protein geranylgeranyltransferase Typ I (GGTase I) (EC 2.5.1.59) combined with substrates GGPP and a protein comprising a cystein, as described herein; protein geranylgeranyltransferase Typ II (RabGGTase) (EC 2.5.1.60) combined with substrates GGPP and a protein comprising a cystein, as described herein; chrysanthemyl diphosphate synthase (EC 2.5.1.67) combined with substrates 2 DMAPP; (2Z,6E)-farnesyl diphosphate synthase (EC 2.5.1.68) combined with substrates GPP and IPP; lavandulyl diphosphate synthase (EC 2.5.1.69) combined with substrates 2 DMAPP; naringenin-8-dimethylallyltransferase (EC 2.5.1.70) combined with substrates (-)-(2S)-naringenin and DMAPP; leachianon-G2"-dimethylallyltransferase (EC 2.5.1.71) combined with substrates leachianon G and DMAPP; 1,4-dihydroxy-2-naphtoat polyprenyltransferase (EC 2.5.1.74) combined with substrates all-trans-polyprenyl diphosphate and 1,4-dihydroxy-2-naphtoat; 7-dimethylallyltryptophan synthase (EC 2.5.1.80) combined with substrates DMAPP and L-tryptophan; geranylfarnesyl diphosphate synthase (EC 2.5.1.81) combined with substrates GGPP and IPP; hexaprenyl diphosphate synthase (EC 2.5.1.82) combined with substrates GGPP and 2 IPP; hexaprenyl diphosphate synthase (EC 2.5.1.83) combined with substrates FPP and 3 IPP; all-trans-nonaprenyl diphosphate synthase (EC 2.5.1.84) combined with substrates GPP and 7 IPP; all-trans-nonaprehyl diphosphate synthase (EC 2.5.1.85) combined with substrates GGPP and 5 IPP; trans,polycis-decaprenyl diphosphate synthase (EC 2.5.1.86) combined with substrates FPP and 7 IPP; ditrans,polycis-polyprenyl diphosphate synthase (EC 2.5.1.87) combined with substrates (2E,6E)-FPP and n IPP (n= 10-55); trans,polycis-polyprenyl diphosphate synthase (EC 2.5.1.88) combined with substrates (2Z,6E)-FPP and n IPP (n= 9-11); tritrans,polycis-undecaprenyl-diphosphate synthase (EC 2.5.1.89) combined with substrates GGPP and 7 IPP; octaprenyl diphosphate synthase (EC 2.5.1.90) combined with substrates FPP and 5 IPP; decaprenyl diphosphate synthase (EC 2.5.1.91) combined with substrates FPP and 7 IPP; (2Z,6Z)-farnesyl-diphosphate synthase (EC 2.5.1.92) combined with substrates DMAPP and 2 IPP; and 4-hydroxybenzoate geranyltransferase (EC 2.5.1.93) combined with substrates GPP and 4-hydroxybenzoate. Numbers in the context of the substrates indicate that a number >1 of substrate molecules is coupled.

In one aspect of the prenylation method of the present invention, the prenyltransferase is selected from isoprenylpyrophosphate synthases, wherein the first substrate is selected from isopentenyl pyrophosphate (IPP), dimethyl allyl pyrophosphate (DMAPP), geranyl pyrophosphate (GPP), farnesyl pyrophosphate (FPP), and geranylgeranyl pyrophosphate (GGPP), and wherein the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP).

In step (b) coupling by an isoprenylpyrophosphate synthase may include coupling of more than one molecule of the first substrate. For example, 2 to 11 or more first substrate molecules, in particular isopentenyl pyrophosphate molecules may be condensed with the second substrate, for example 2, 3, 4, 5, 6, 7, 8, 9, 10, 11, 9 to 11, 10 to 55 or more first substrate molecules, in particular isopentenyl pyrophosphate moieties.

In step (b), coupling of 1, 2, 3, 4, 5, 7, 9 to 11, or 10 to 55 isopentenyl pyrophosphate molecules to the second substrate by an isoprenylpyrophosphate synthase is preferred.

Coupling by an isoprenylpyrophosphate synthase may result in a compound selected from hexaprenyl pyrophosphate (C₃₀), heptaprenyl pyrophosphate (C₃₅), octaprenyl pyrophosphate (C₄₀), nonaprenyl pyrophosphate (C₄₅) and decaprenyl pyrophosphate (C₅₀).

In another aspect of the prenylation method of the present invention, the prenyltransferase is selected from protein prenyltransferases, and the first substrate is selected from proteins, and the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP). Preferably, the second substrate is selected from farnesyl pyrophosphate and geranylgeranyl pyrophosphate.

Coupling of the second substrate to the protein preferably takes play via a thioether linkage (-C-S-C-) to a cysteine. The cystein may be located at or near the carboxyl terminus of the protein. In this context "near the carboxyl terminus" in particular refers to a cysteine located at a position located 1, 2, 3, 4, 5, 6, 7, 8, 9, 10, at the maximum 15, at the maximum 20, or at the maximum 30 amino acid residues distant from the carboxy terminus.

In yet another aspect of the prenylation method of the present invention, the prenyltransferase is selected from aromatic prenyltransferases, and the first substrate is selected from compounds comprising an aromatic group, and the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP).

Further, the present invention shall be further illustrated by the following figures and example:
- **Figure 1:**: Translocation mechanism of autotransporter proteins. The N-terminal signal peptide facilitates the transport across the inner membrane and is then cut off by periplasmic signal peptidases. The C-terminal part forms a β-barrel structure inside the outer membrane through which the passenger is translocated to the surface by the linker.

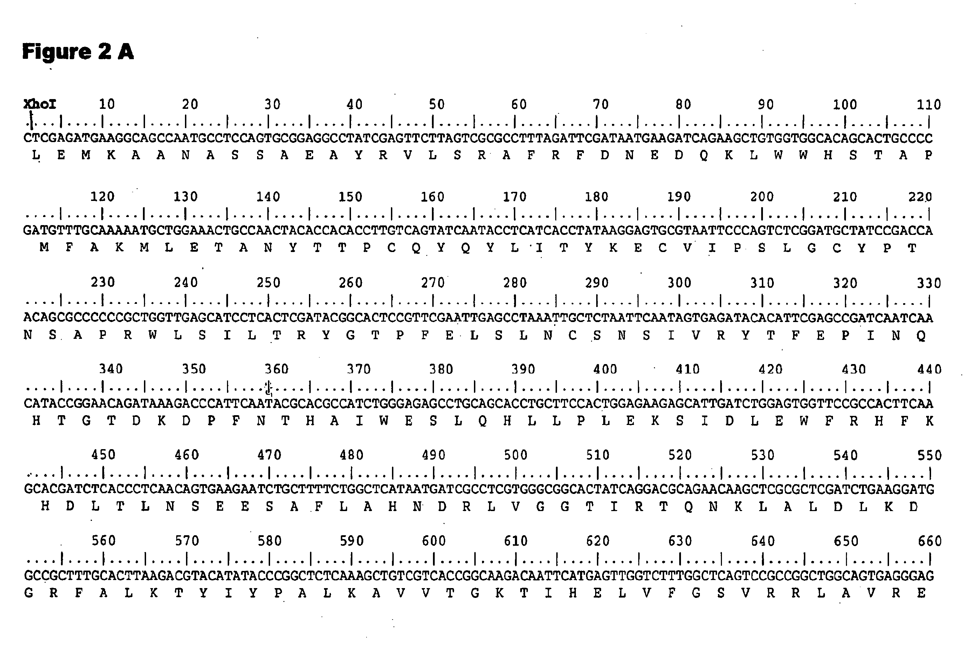

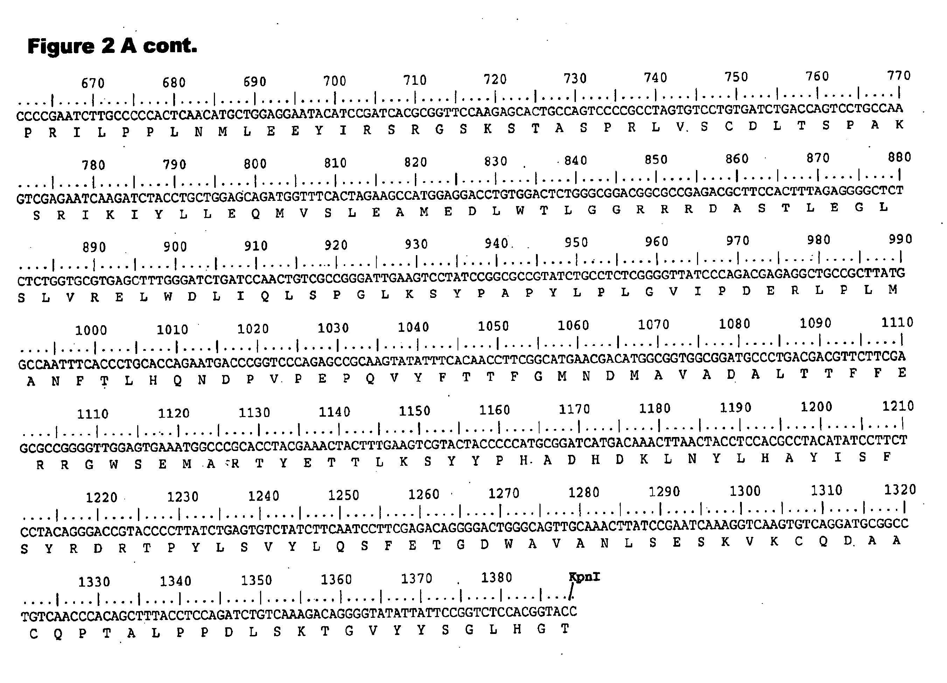

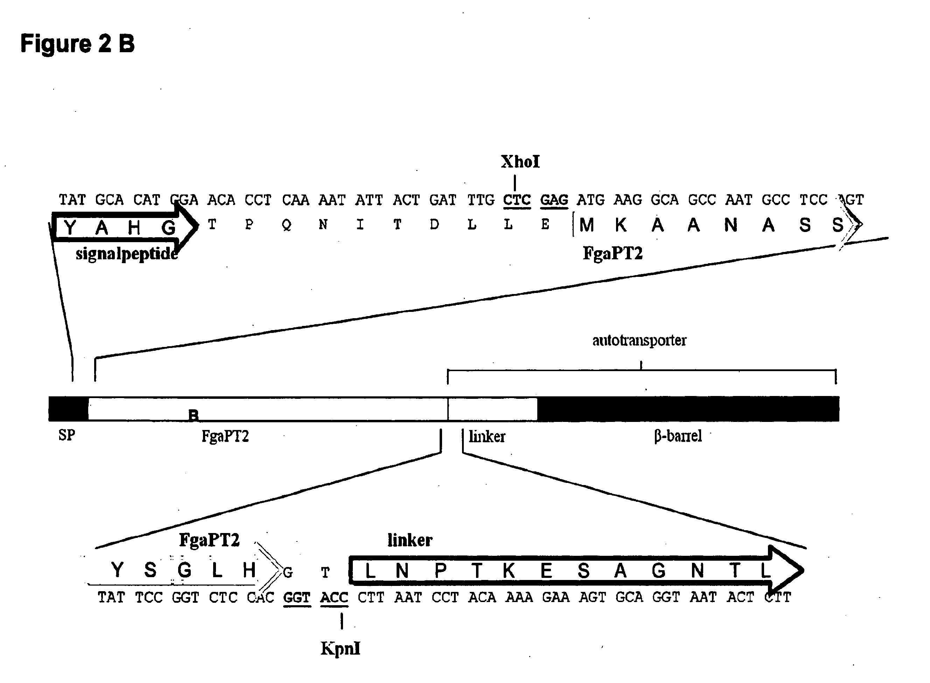

- **Figure 2:**: **A:** Nucleotide sequence (SEQ ID NO:1) and deduced amino acid sequence (SEQ ID NO:2) of the fgaPT2-PCR-product. Sites for the restriction endonucleases are underlined. B: The structure of the precursor protein FgaPT2-AT encoded by pEK004. The surrounding of the fusion sites is given as nucleotide and amino acid sequences. The restriction sites used for cloning are underlined. The N- and C-terminal sequence of the FgaPT2 passenger is indicated by a white arrow. The C-terminus of the signal peptide is represented by the dark grey arrow and the light grey arrow depicts the N-terminus of the linker region.

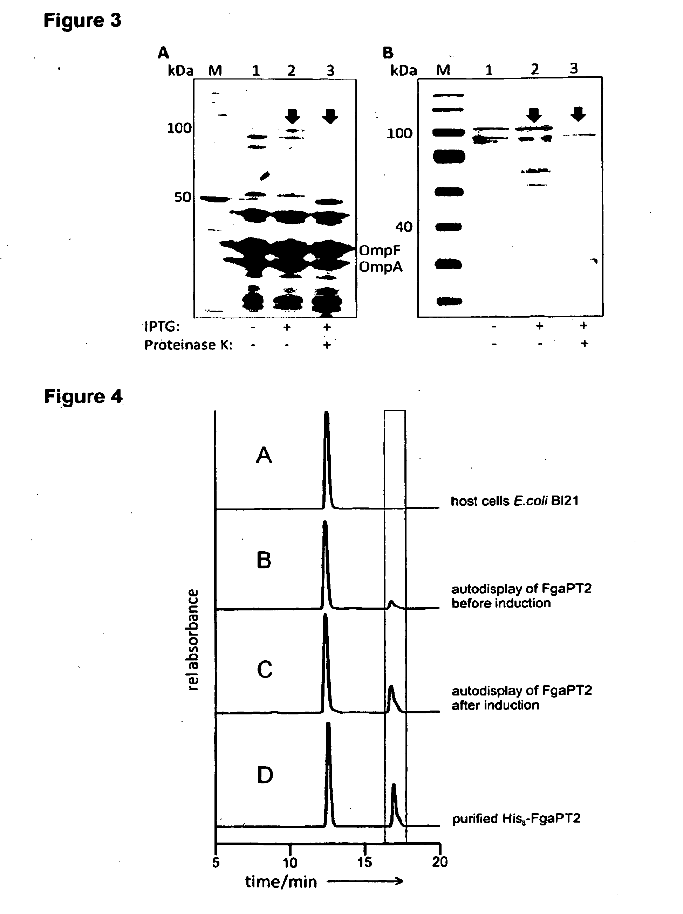

- **Figure 3:**: Surface display of FgaPT2-AT-fusion protein. SDS-PAGE (A) and Western Blot analysis (B) of outer membrane protein preparations from E. *coli* BL21 (DE3) pEK004 (lane 1-3). Molecular weight markers are indicated at the left. M: protein marker, IPTG+/-: protein expression was induced by adding IPTG at a final concentration of 1 mM. Proteinase K +/-: Whole cells were treated with Proteinase K. OmpA/F/C: *E*. *coli* natural outer membrane proteins. The FgaPT2-AT-fusion protein is indicated by the black arrows.
- **Figure 4:**: HPLC chromatograms of the assays using the FgaPT2 whole cell biocatalyst before (B) or after induction (C). Host cells (A) and purified enzyme His8-FgaPT2 (D) were used as controls.

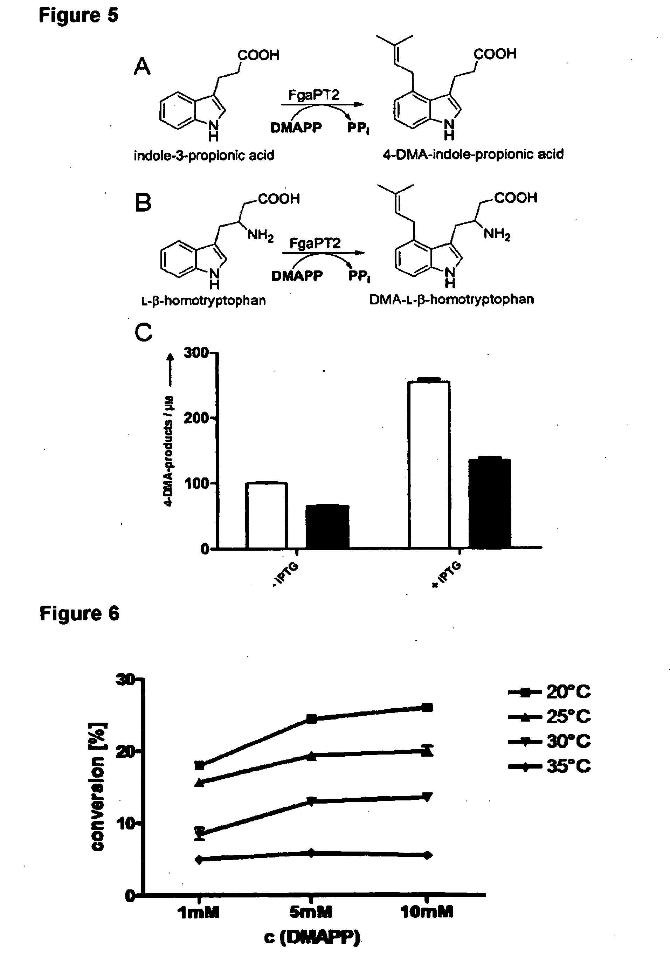

- **Figure 5:**: Prenylation of indole-3-propionic acid (A) and L-β-homotryptophan (B) at C4 resulting in 4-dimethylallyl-propionic acid respectively 4-dimethylallyl-tryptophan. C: Enzymatic activity of whole cells expressing FgaPT2. Conversion of indole-3-propionic acid (white columns) and L-β-homotryptophan (black columns) before and after induction with IPTG was analyzed. The reaction was run for 24 hours in a 100 µl sample volume with an OD₅₇₈= 40. Reaction products were determined by HPLC of the supernatant after removal of the cells by centrifugation.
- **Figure 6:**: Dependence of product formation on different assay conditions. FgaPT2 activity of whole cells was analyzed at different temperatures and different concentrations of DMAPP. Conversion rates of indole-3-propionic acid at 20 °C [■], 25 °C [▲], 30 °C [▼] and 35 °C [◆] are shown. The reaction product was determined by HPLC. Mean value and standard deviation are presented, (n= 3).

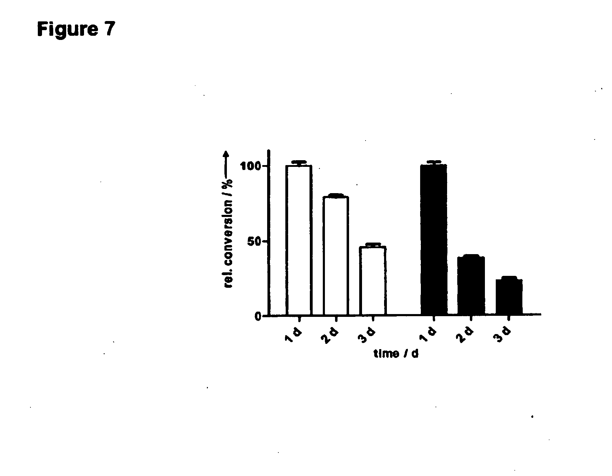

- **Figure 7:**: Reusability of the whole cell biocatalyst for 3 reaction cycles. Conversion of indole-3-propionic-acid (white columns) and L-β-homotryptophan (black columns) was tested. Conversion was determined by HPLC after 24 hours of incubation for each cycle. The relative rate of conversion is presented by defining the first conversion as 100 %.

### Example: Whole cell biocatalyst for the efficient prenylation of indolederivatives by autodisplay of the aromatic prenyltransferase FgaPT2

### Summary

The following study depicts the development of a whole cell biocatalyst for the prenylation of indole derivatives. For this purpose the prenyltransferase FgaPT2 from *Aspergillus fumigatus* was displayed on the surface of *Escherichia coli* cells by using Autodisplay. The presence of the prenyltransferase in the outer membrane was detected by SDS-PAGE and Western Blot after the proteins of the outer membrane were isolated. The orientation of the prenyltransferase towards the outside of the cells was investigated by accessibility testing with externally added proteases. The FgaPT2 whole cell biocatalyst converted up to 250 µM of indole-3-propionic acid, approximately 25 % of the substrate used in the assay (100 µL sample, OD₅₇₈= 40). Another indole substrate, L-β-homotryprophan was also investigated and a conversion of 13 % was determined. By optimizing the assay conditions the conversion rate could be raised to approximately 30 % of indole-3-propionic acid during a 24 h incubation time at 20 °C. The whole cell biocatalyst endured a storage period of one month at 8 °C without any detectable loss in activity. Reusability was confirmed. After three cycles of consecutive use the whole cell biocatalyst retained a conversion rate of 46 % of indole-3-propionic acid and 23 % of L-β-homotryptophan after the third cycle.

### Results

### Construction of a dimethylallyl-transferase- autotransporter fusion protein

The *fgaPT2* gene was amplified by PCR from plasmid pIU18 [16]. The PCR primers added an Xhol site at the 5' end and a KpnI site at the 3' end of *fgaPT2* (SEQ ID NOs:1 and 2 in Figure 2 A). The PCR fragment was inserted into pCR4^{®}-TOPO^{®}-vector and recleaved with XhoI and KpnI. The restriction fragment was ligated into pET-Adx04, which had also been cleaved with the same enzymes. Insertion of the *fgaPT2* PCR fragment resulted in plasmid pEK004 which encodes an artificial fusion protein composed of an N-terminal signal sequence derived from choleratoxin β-subunit, FgaPT2 as a passenger and the C-terminal AIDA-I autotransporter region (Figure 2 B). Within this autotransporter a linker and a β-barrel structure cause the translocation across the outer membrane (Figure 1). Plasmid pEK004 was transformed into *E. coli* BL21(DE3). Expression of the autotransporter fusion protein was induced by addition of IPTG to a final concentration of 1 mM for one hour at 30 °C and 200 rpm. Isolates of outer membrane proteins were analyzed by SDS-PAGE. A basal expression of fusion protein occurred even in the absence of IPTG (Figure 3A and B, lane 1). This is presumably due to the described leakiness of the *lac*UV promoter [26].

### Surface display of FgaPT2

To prove whether surface exposure occurred effectively, the accessibility of the fusion protein for proteases was tested. To do this, protein expression was induced and whole cells were subsequently treated with Proteinase K. Since proteases are too large to cross the outer membrane, proteins can only be digested by proteases when exposed at the surface. Treatment of whole cells with Proteinase K resulted in digestion of the fusion protein (Figure 3). To demonstrate whether the outer membrane stays intact during the proteinase accessibility test, OmpA can be used as an integrity reporter for the outer membrane. The C-terminal part of OmpA directs to the periplasmic space between the inner and outer membrane. The N-terminal part builds a β-barrel structure inside the outer membrane but does not point to the outside [27]. Thus OmpA is only sensitive against digestion from the periplasmic side but not from the outside and therefore indicates membrane integrity. If a protease is added and only the fusion protein but not OmpA is digested, integrity of the cell is assured and the fusion protein can be regarded as surface exposed. Since OmpA was not digested by Proteinase K during our experiment, the accessibility test provided strong proof of surface exposure (Figure 3A, lane 3). We did notice a slight difference in intensity of the protein bands of OmpA as well as of OmpF (lane 3) compared to the non digested sample (lane 2). This might be due to a minor difference in protein concentration. The Western Blot prepared from the same samples (Figure 3 B) shows a clear decrease in intensity of the fusion protein due to Proteinase K treatment.

### Enzyme activity of surface displayed FgaPT2

After confirming the surface exposure of FgaPT2, the cells displaying FgaPT2 were tested for enzymatic activity. Cells were prepared as described above and conversion of 1 mM indole-3-propionic acid and L-β-homotryptophan was carried out for 24 hours in 100 µL assay samples. Reaction products were analyzed by HPLC. Host cells *E. coli* BL21(DE3) without a plasmid, used as a negative control, did not show dimethylallyltransferase activity (Figure 4 A).

Cells harbouring the plasmid encoding for the autotransporter fusion protein showed dimethylallyltransferase activity even without induction of protein expression (Figure 4 B). This was presumably due to a leakiness of the *lac*UV promoter, a common effect that causes a basal expression of active fusion protein. After induction of protein expression, cells displaying the fusion protein clearly showed higher activity (Figure 4 C). The retention time of reaction products of conversion with whole cells displaying the fusion protein on the surface corresponded very well to the retention time of reaction product yielded with purified enzyme His₈-FgaPT2 which was used as a positive control (Figure 4 D).

Conversion rates were then quantified. It was calculated, that 100 µM of 4-dimethylallyl(DMA)-indole-3-propionic acid, respectively 64 µM of 4-DMA-β-homotryptophan were formed in samples before induction of protein expression (Figure 5 C). Samples after induction of protein expression contained 254 µM of 4-DMA-indole-3-propionic acid and approximately 133 µM formation of 4-DMA-β-homotryptophan (Figure 5 C).

### Identification of the enzymatic products by LC-MS

The corresponding mass of the enzymatic products yielded with the whole cell biocatalyst was determined by mass spectrometry. The obtained fragmentation pattern (Table 1) was in good accordance with the data for 4-DMA-indole-3-propionic acid and 4-DMA-β-homotryptophan published previously [16]. It has already been shown in a previous study, that the prenylation by FgaPT2 takes place regiospecifically at position 4 of the indole ring [15-16].

**Table 1:**

| **MS data of the 4-prenylated products (m/z, relative intensity)** | | |
|---|---|---|
| | 4-DMA-indole-3-propionic acid | 4-DMA-ß-homotryptophan |
| Mr | 257.14 | 286.17 |
| [M+1]+ | - | 287.2 |
| [2M+1]+ | - | 573.1 |
| ms²[M+1]+ (relative intensity) | - | 270.1 (23), 252.2 (6), 231.1 (11), 219.1 (4), 214.1 (100), 210.2 (16), 200.2 (15) |
| [M-1]- | 256.3 | 285.5 |
| [2M-1]- | 513.8 | 571.4 |
| ms²[M-1]-(relative intensity) | 256.0 (100), 212.1 (9), 210.2 (4), 184.1 (49) | 285.1 (100), 241.2 (4), 225.2 (6), 224.2 (19) |

### Influence of incubation temperature and DMAPP concentrations on the enzymatic reaction

To optimize enzymatic activity of the surface displayed FgaPT2 incubation temperature and concentration of DMAPP cosubstrate were modified. As shown in Figure 6, the highest conversion of substrate was achieved at 20 °C. If the concentration of cosubstrate additionally was raised from 1 mM to 5 mM and 10 mM the conversion rate at 20 °C increased from 20 % to almost 30 %. Compared to that, incubation at 35 °C only achieved less than 10 % of substrate conversion. At these conditions increasing concentrations of cosubstrate did not result in higher conversion rates. This was presumably due to the fact that the temperature and not concentration of cosubstrate was the limiting factor. This effect could be observed when conversion was carried out at 25 °C and 30 °C.

Raising DMAPP concentrations from 1 mM up to 5 mM caused a further increase in substrate conversion from 8 to 13 % at 25 °C and from about 15 to 20 % at 30 °C respectively. At these temperatures enzymatic activity of surface displayed FgaPT2 is higher, but 5 mM of cosubstrate was sufficient to sustain the enzymatic reaction for 24 hours. In summary, the optimum enzymatic activity of surface displayed FgaPT2 was at 20 °C incubation temperature and 10 mM cosubstrate concentration.

### Reusability of the FgaPT2 whole cell biocatalyst

One of the advantages of whole cell biocatalysts is their simple availability and the possibility to easily recover the cells from the reaction mixture. Therefore the cells need to be sufficiently stable to endure the recovery treatment. Hence the reusability of the FgaPT2 whole cell biocatalyst should be analyzed in three consecutive activity test cycles. Assays were carried out with 1 mM indole-3-propionic acid or L-β-homotryptophan and 1 mM of DMAPP cosubstrate at 20 °C. After 24 hours of incubation, the cells were removed by centrifugation. The supernatant was analyzed by HPLC. The cells were resuspended in buffer, substrates were added and the assays were again incubated at 20 °C. Procedure was repeated every 24 hours for 3 days. In comparison to the first day, the enzyme retained 46 % and 23 % of its activity in the production of 4-DMA-indole-propionic-acid and DMA-β-homotryptophan, respectively (Figure 7).

### Long term stability of stored whole cell biocatalysts

In order to determine long term stability of the whole cell biocatalyst, a suspension of induced cells (OD₅₇₈= 50) was stored at 4 °C for 1 month. To avoid sedimentation of cells, the suspension was mixed every 24 hours by inversion of the tubes several times. Before and after one month of storage, activity was determined. Compared to the conversion of substrate measured before storage, no decrease in enzymatic activity of stored cells was observed (Table 2). The number of viable cells in the freshly prepared and stored samples was also determined. Although there was no decrease in activity, the number of viable cells in one assay sample differed 10 fold from 4.2 million cells/100 µL in the fresh sample to 400.000 cells/100 µl in the stored one.

**Table 2:**

| **Long term stability of the stored whole cell biocatalyst** | |
|---|---|
| Storage time | indole-3-propionic-acid^{[a]} |
| Fresh | 254.0 ± 0.40 |
| 1 month | 264.8 ± 0.02 |
| [a] Mean value of conversion [µM] ± SD | |

### Efficiency of the whole cell biocatalyst

The number of enzyme molecules displayed via Autodisplay was formerly calculated as about to be 10-20x 10⁵[22]. Since the number of viable cells of the FgaPT2 displaying whole cell biocatalyst was determined, the number of enzyme molecules in one assay sample containing the whole cell biocatalyst could be calculated. Regarding the fact that the enzyme is expressed as a monomer and needs to form active dimers on the cell surface, the number of functional surface displayed enzymes sums up to 2-4x10¹¹ (q.v. Table 3). The number of purified His8-FgaPT2 enzyme molecules in the purified enzyme control was determined to be 6x10¹². Hence the sample with purified His₈-FgaPT2 contained one order of magnitude more enzyme molecules than the sample with the whole cell biocatalyst. Based on these findings a direct determination of efficiency of the FgaPT2 whole cell biocatalyst was possible. We then compared the conversion rates of indole-3-propionic acid in both assays. Although the number of enzyme molecules was lower, the whole cell biocatalyst reached nearly 75 % of the conversion yielded by the purified enzyme during the same reaction time. Both conversions, with whole cell biocatalyst and purified enzyme respectively, were carried out for 24 h. For the purified enzyme a conversion of 32 % of the used substrate indole-3-propionic acid within one hour of reaction time is reported [16]. Increasing the reaction time to 24 h did not greatly improve the yield of converted substrate (34 %), suggesting that the enzyme is unstable and rapidly loses activity. In comparison the present study shows that an efficient and obviously more stable application of FgaPT2 could be achieved by Autodisplay of FgaPT2 and employment of whole cells for substrate conversion.

**Table 3:**

| **Efficiency of the FgaPT2-whole cell biocatalyst compared to purified enzyme** | | |
|---|---|---|
| | Purified enzyme | Whole cell biocatalyst |
| Enzyme molecules per sample | 6*10¹² | 2-4*10¹¹ |
| Substrate conversion [%]^{[a]} | 34 | 25,4 |
| Conversion rate [%] | 100 | 74,7 |
| Relative conversion per enzyme molecule [fmol] | 5.66*10⁻⁶ | 1,27*10⁻⁴ |
| [a] 1m_{M} start concentration of indole-3-propionic acid | | |

### Discussion

Within this Example we used Autodisplay for the development of a functional whole cell biocatalyst for the synthesis of prenylated indole derivatives. The attachement of a prenyl moiety to an aromatic acceptor often increases the biological effect of the molecule. C₈-prenylation of the flavonoids apigenin and liquiritigenin dramatically enhances their cytotoxic effect compared to their non-prenylated precursors [9]. Tryprostatins, prenylated derivatives of *cyclo*-L-Trp-L-Pro derived from *A. fumigatus,* showed cell cycle inhibiting effects [28]. One diastereomer of tryprostatin B exhibited an even more potent cytotoxicity profile than etoposide when it was tested in human prostate, lung and breast cancer cell lines [29]. Hence the prenylation step is of tremendous importance not only during the biosynthesis of cytotoxically active second metabolites in plants and fungi but most likely also in a chemoenzymatic synthesis of possible anti tumor agents. Even though the total synthesis of tryprostatin A and B has already been demonstrated [30], employment of a biocatalyst for prenylation offers the advantages of lower energy consumption, regiospecific attack and less bothersome by-products, since the prenylation step in the chemical synthesis of tryprostatins needs to take place under the use of isoprenylbromide and at -78 °C. The biocatalyst also offers the opportunity for synthesis of a variety of active compounds and intermediates, because aromatic prenyltransferases show a low substrate specificity and are thus able to accept a rather broad diversity of substrates [7,13].

When incubated with indole-3-propionic acid as a substrate for 24 h, cells displaying FgaPT2 on their surface showed a conversion of about 25 % of the deployed substrate after protein expression was induced by adding IPTG. In cells which were not treated with IPTG, a conversion of 10 % of indole-3-propionic acid was measured. It is a reasonable assumption that this activity is caused by a basal expression of the desired protein, caused by leakiness of the *lac*UV promoter that controls T7 polymerase expression in the applied *E. coli* strain BL21(DE3) [33]. The conversion of a second indole substrate, L-β-homotryptophan, within 24 h by the whole cell biocatalyst was determined to be 13 % after induction and 6 % before induction. It was not possible to determine conversion of the native substrate tryptophan, since it is already completely converted into another not prenylated product by the host cells *E. coli* BL21(DE3) and some other expression strains that we tested (*E. coli* UT5600(DE3), data not shown). While not wishing to be bound by theory, this could be explained by a slower prenylation of tryptophan than conversion of tryptophan into other not prenylated products in these host cells. Higher concentrations of tryptophan may saturate these pathways, so that tryptophan prenylation could be observed.

The FgaPT2 whole cell biocatalyst showed the highest activity (30 % of substrate converted) at 20 °C and addition of 10 mM of DMAPP cosubstrate. These can be considered as very mild conditions, because a high conversion rate was yielded even at room temperature instead of 30 °C which was the temperature optimum for the purified enzyme [15]. Furthermore, the functional expression of FgaPT2 provides further evidence for the motility of the β-barrels in the outer membrane. Since FgaPT2 is supposed to be active only in a dimeric state [14], our results suggest that dimerisation occured on the outer membrane. This effect of spontaneous dimerisation has already been shown for the Autodisplay of bovine adrenodoxin [22] and sorbitol dehydrogenase [23]. Even higher aggregates could be functionally expressed by Autodisplay, as shown for a multihomomeric nitrilase from *Alcaligenes faecalis* [24].

In applying the FgaPT2-whole cell biocatalyst in three consecutive activity assays with recovery of the catalyst via centrifugation, the whole cell biocatalyst was reusable and after three cycles retained 46 % and 23 % of its activity towards indole-3-propionic acid and L-β-homotryptophan respectively. The loss of activity may be due to a loss of cells during the centrifugation and resuspension steps. It is also possible that a certain amount of surface displayed protein suffers from abrasion during the treatment since a relatively high optical density (OD₅₇₈=40) was used, possibly resulting in mechanical stress of the surface displayed proteins.

The storage of cell samples for 1 month at 8 °C was also possible without any loss of activity. After one month of storage the number of viable cells decreased to 10 % of the original sample, however, the stored cells showed the same conversion rate as a fresh sample. The herein tested viability only describes the cells ability to reproduce. The measured loss of viability therefore is not equivalent with death or lysis of cells. As long as the cell or the outer membrane remains intact respectively, the surface displayed enzymes still can be active. Storage at -20 °C or -70 °C was not tested but also is theoretically possible. Storage stability at -70 °C was proven to be possible for a whole cell biocatalyst displaying a nitrilase from *A. faecalis* by means of Autodisplay [24].

When compared to conversions of indole-3-propionic acid by the purified enzyme, the whole cell biocatalyst showed its effectiveness. The conversion of indole-3-propionic acid of the purified enzyme was 34 % in 24 h. During one hour of reaction time 32 % of indole-3-propionic acid were converted, whereas the native substrate tryptophan was converted completely [16]. Regarding the number of enzymes accomplishing this conversion in an assay sample (Table 3) there was one order of magnitude more molecules of purified enzyme within one sample than enzyme molecules on the surface of the whole cell biocatalyst. Taking into account that there was no further increase in substrate conversion by the purified enzyme when the reaction time was extended, it can be assumed that the enzyme is instable and rapidly loses its activity. So with a substrate conversion of 25 % by the whole cell biocatalyst, nearly 75 % of conversion rate yielded by the purified enzymes were reached with less molecules of enzymes, suggesting that Autodisplay increases the stability of FgaPT2.

In this Example we demonstrated the capability of Autodisplay for the development of a functional whole cell biocatalysts. The surface displayed prenyltransferases showed excellent performance towards the tested substrates and a high efficiency of conversion under very mild conditions. By immobilizing the enzyme in the outer membrane of *E. coli* its stability was improved enormously. As the purified prenyltransferase is reported to act on the dipeptide precursor molecules of tryprostatins [17], these molecules can be used as substrates for the whole cell biocatalyst. The use of surface-displayed prenyltransferases in the syntheses of tryprostatins would be a significant step forward in the chemoenzymatic synthesis of anti cancer agents.

### Experimental section

**Bacterial strains, plasmids and culture conditions:** *Escherichia coli* strains UT5600(DE3) [F⁻, *ara*-14, /*eu*B6, secA6, *lac*Y1, *pro*C14, *tsx*-67, Δ(*omp*T-fepC)266, *ent*A403, *trp*E38, *rfb*D1, *rps*L109(Str^{r}), *xyl*-5, *mtl*-1, *thi*-1, λ(DE3)] and BL21(DE3) [B, F⁻, *dcm, omp*T, *Ion, hsdS(rB-* mB-), *gal*, λ(DE3)] were used for the expression of autotransporter fusion proteins. *E.coli* TOP10 (F- *mcrA Δ*(*mrrhsd*RMS-*mcr*BC) Φ80/*ac*ZDM15 Δ*lac*X74 deoR recA1 *ara*D139 Δ(*ara-leu*) 7697 *gal*U *gal*K *rps*L (Str^{R}) *end*A1 *nup*G) and the vector pCR^{®}4-TOPO^{®} which were used for subcloning of PCR products were obtained from Invitrogen. Plasmid pET-Adx04 which encodes the AIDA-I autotransporter and plasmid pHis8-PT2 (pIU18) which encodes dimethylallyltryptophansynthase FgaPT2 have been described elsewhere [16,22]. Bacteria were routinely grown at 37°C in Lysogeny broth (LB) containing ampicillin (100mgL⁻¹). For FgaPT2 expression studies and enzyme assays cells were cultured in the presence of ethylendiamintetraacetate (EDTA, 10 µM) and 2-mercaptoethanol (10 mM).

**Recombinant DNA techniques:** For construction of plasmid pEK004, which contains the gene encoding the FgaPT2-autotransporter fusion protein, the *fgaPT2* gene was amplified by PCR. Plasmid pHis8-PT2 (pIU18) was used as template for primers EK003 (5'- CTGCTCGAGATGAAGGCAGCCAATGCCTC -3', SEQ ID NO:5) and EK004 (5'- GGGGTACCGTGGAGACCGGAATAATATACC -3', SEQ ID NO:6). The PCR product was inserted into vector pCR^{®}4-TOPO^{®} and recleaved with XhoI and KpnI. The restriction fragment was ligated to pET-Adx04, restricted with the same enzymes. This yields an in frame fusion of *fgaPT2* with the autotransporter domains under the control of a T7/lac promoter (Figure 2) as described by Petermann *et al.* [34]. Preparation of plasmid DNA, ligation, restriction digestion, transformation procedures and DNA electrophoresis were performed according to standard protocols [35].

**Outer membrane preparation:** *E. coli* cells were grown overnight and 1 ml of the culture was used to inoculate LB medium (40 ml). Cells were cultured at 37 °C with vigorous shaking (200 rpm) for about 2 hours until an OD₅₇₈ of 0.5 was reached. The culture was separated into two aliquots and protein expression was induced by adding IPTG at a final concentration of 1 mM to one of the aliquots. Cultures then were incubated at 30 °C and shaking (200 rpm) for one hour. Induction was stopped by incubating the cells on ice for 15 min. After harvesting and washing of the cells with Tris-HCl (0.2 M, pH 8), differential cell fractionation was performed according to the method of Hantke [36] as modified by Schultheiss *et al.* [36-37]. The outer membrane proteins were washed, resuspended in water and prepared for SDS-PAGE. For whole cell protease treatment, *E. coli* cells were harvested, washed and resuspended in 1 ml Tris-HCl (0.2 M, pH 8). Proteinase K was added to a final concentration of 0.2 mg ml⁻¹ and cells were incubated for 1 hour at 37 °C. Digestion was stopped by washing the cells twice with Tris-HCI (0.2 M, pH 8) containing 10 % fetal calf serum (FCS) and outer membrane proteins were prepared as described above.

**SDS-PAGE and Western Blot Analysis:** Outer membrane isolates were diluted (1:1.5) with sample buffer (100 mM Tris/HCl (pH 6.8) containing 4 % SDS, 0.2 % bromophenol blue, 200 mM dithiothreitol and 20 % glycerol), boiled for 5 minutes and analyzed on 10 % SDS-PAGE. Proteins were stained with Coomassie brilliant blue (R250). For Western Blot analysis, gels were electroblotted on polyvinylidenefluoride (PVDF) membranes. Blotted membranes were blocked in TBS with 3 % bovine serum albumin (BSA). For immune detection, membranes were incubated with the primary anti-FgaPT2 polyclonal antibody serum diluted 1:2000 in TBS with 3 % BSA for 3 hours. Prior to addition of the secondary antibody, immunoblots were rinsed three times in TBS with 0.1 % Tween 20. The secondary antibody was added and the blots were incubated for 2 hours at room temperature. Antigen-antibody conjugates were visualized by treatment with horseradish peroxidase linked goat anti-rabbit IgG diluted 1:5000 in TBS with 3% BSA. A colour reaction was achieved by adding a solution consisting of 4-chloro-1-naphthol (3mg mL⁻¹) in methanol (5mL), TBS (25mL), and H₂O₂ (30 %,15µL).

**Chemicals for prenyltransferase assay:** Trisammonium salt of DMAPP was synthesised in analogy to trisammonium geranyl diphosphate synthesis reported by Woodside *et al.* [38]. L-β-homotryptophan was purchased from Fluka (Seelze, Germany) and indole-3-propionic acid from Aldrich (Seelze, Germany).

**Conditions for enzymatic reactions:** For determination of enzymatic activity of surface displayed FgaPT2, reaction products were measured by HPLC. For this purpose cells were grown over night and then used 1:100 to inoculate a fresh culture. At an OD₅₇₈ of 0.5, protein expression was induced by adding 1 mM of IPTG and cells were incubated for 4 hours at 30 °C and 200 rpm. After induction cells were washed twice with Tris-HCl 50 mM pH 7.5, resuspended in the same buffer to an OD₅₇₈ of 50 and stored on ice. The assay with His₈-FgaPT2 was carried out as described previously [16]. The standard assay for determination of activity of the displayed FgaPT2 (100 µl) was carried out in Tris-HCl 50 mM pH 7.5 and contained 1 mM tryptophan derivative, 1 - 10 mM DMAPP and 80 µl cell suspension (OD₅₇₈= 40). The reaction mixtures were incubated for 24 h at 20 - 35 °C. After removal of the cells by centrifugation for 10 min at 14.000 x g and -4 °C, the supernatant was transferred into a new tube and protein was precipitated by 1 volume methanol. After centrifugation, the supernatant was analyzed by HPLC and LC-MS as described below. Two independent assays were carried out for quantification.

**HPLC analysis:** Reaction mixtures were analyzed on an HP HPLC Series 1090 by using a Multospher 120 RP18 (5 µm, 250 x 4 mm) at a flow rate of 1 ml•min⁻¹. Water (solvent A) and acetonitrile (solvent B), each containing 0.5 % trifluoroacetic acid, were used as solvents. A gradient was run from 15 % to 70 % B in 13 min. After washing with 100 % solvent B for 5 min, the column was equilibrated with 15 % solvent A for 5 min. The substances were detected with a Photo Diode Array detector and illustrated for absorption at 296 nm.

**Spectroscopic analysis:** The assays were analyzed by positive and negative electrospray ionization (ESI) mass spectrometry with a ThermoFinnigan TSQ Quantum. The mass spectrometer was coupled with an Agilent HPLC series 1100 equipped with a RP18-column (2 x 250 mm, 5 µm). For separation, the column was run with 10 % solvent B (methanol) in solvent A (water, each containing 0.1 % HCOOH) for 5 min, followed by a gradient from 10 % to 100 % B over 30 min. After washing with 100 % B, the column was equilibrated with 10 % B for 10 min. The flow rate was at 0.2 ml•min⁻¹.

### References

[1] P. H. Liang, T. P. Ko, A. H. J. Wang, Eur. J. Biochem. 2002, 269, 3339-3354.
[2] C. D. Poulter, J. Agric. Food Chem. 1974, 22, 167-173.
[3] C. D. Poulter, H. C. Rilling, in Biosynthesis of Isoprenoid Compounds, Vol. 1 (Ed.: S. L. Spurgeon), John Wiley & Sons, New York, 1981, pp. 225- 282.
[4] J. A. Glomset, C. C. Farnsworth, Annual Review of Cell Biology 1994, 10, 181-205.
[5] W. R. Schafer, J. Rine, Annu. Rev. Genet. 1992, 26, 209-237.
[6] L. Heide, Curr. Open. Chem. Biol. 2009, 13, 171-179.
[7] S. M. Li, Natural product reports 2010, 27, 57-78.
[8] B. Botta, A. Vitali, P. Menendez, D. Misiti, G. D. Monache, Curr. Med. Chem. 2005, 12, 713-739.
[9] W. Wätjen, N. Weber, Y. j. Lou, Z. q. Wang, Y. Chovolou, A. Kampkötter, R. Kahl, P. Proksch, Food Chem. Toxicol. 2007, 45, 119-124.
[10] B. Botta, G. D. Monache, P. Menendez, A. Boffi, Trends Pharmacol. Sci. 2005, 26, 606-608.
[11] J. F. Sanz-Cervera, E. M. Stocking, T. Usui, H. Osada, R. M. Williams, Bioorg. Med. Chem. 2000, 8, 2407-2415.
[12] C. Wallwey, S. M. Li, Natural product reports 2011, 10.1039/C0NP00060D
[13] S. M. Li, Appl. Microbiol. Biotechnol. 2009, 84, 631-639.
[14] U. Metzger, C. Schall, G. Zocher, I. Unsöld, E. Stec, S. M. Li, L. Heide, T. Stehle, Proc. Natl. Acad. Sci. USA 2009, 106, 14309-14314.
[15] I. A. Unsöld, S. M. Li, Microbiology 2005, 151, 1499-1505.
[16] N. Steffan, I. A. Unsold, S. M. Li, ChemBioChem 2007, 8, 1298-1307.
[17] N. Steffan, S. M. Li, Arch. Microbiol. 2009, 191, 461-466.
[18] P. Samuelson, E. Gunneriusson, P. Å. Nygren, S. Ståhl, J. Biotechnol. 2002, 96, 129-154.
[19] J. Jose, T. F. Meyer, Microbiol. Mol. Biol. Rev. 2007, 71, 600-619.
[20] J. Maurer, J. Jose, T. F. Meyer, J. Bacteriol. 1997, 179, 794-804.
[21] J. Jose, Appl. Microbiol. Biotechnol. 2005, 1-8.
[22] J. Jose, R. Bernhardt, F. Hannemann, Chembiochem 2001, 2, 695-701.
[23] J. Jose, S. von Schwichow, Chembiochem 2004, 5, 491-499.
[24] C. Detzel, R. Maas, J. Jose, ChemCatChem, 2011, 3,719-725
[25] E. Schultheiss, S. Weiss, E. Winterer, R. Maas, E. Heinzle, J. Jose, Appl. Environ. Microbiol. 2008, 74, 4782-4791.
[26] F. W. Studier, B. A. Moffatt, J. Mol. Biol. 1986, 189, 113-130.
[27] R. Koebnik, L. Kramer, J. Mol. Biol. 1995, 250, 617-626.
[28] M. Kondoh, T. Usui, T. Mayumi, H. Osada, J. Antibiot. 1998, 51, 801-804.
[29] S. Zhao, K. S. Smith, A. M. Deveau, C. M. Dieckhaus, M. A. Johnson, T. L. Macdonald, J. M. Cook, J. Med. Chem. 2002, 45, 1559-1562.
[30] S. Zhao, T. Gan, P. Yu, J. M. Cook, Tetrahedron Lett. 1998, 39, 7009-7012.
[31] W. A. Duetz, J. B. Van Beilen, B. Witholt, Curr. Opin. Biotechnol. 2001, 12, 419-425.
[32] T. Ishige, K. Honda, S. Shimizu, Curr. Opin. Chem. Biol. 2005, 9, 174-180.
[33] F. W. Studier, J.Mol.Biol. 1991, 219, 37-44.
[34] K. Petermann, S. Vordenbäumen, J. C. Pyun, A. Braukmann, E. Bleck, M. Schneider, J. Jose, Anal. Biochem. 2010, 407, 72-78.
[35] J. Sambrook, E. F. Fritsch, T. Maniatis, Molecular Cloning. A laboratory manual, Cold Spring Harbor Press, 1989**.**
[36] K. Hantke, Mol. Gen. Genet. 1981, 182, 288-292.
[37] E. Schultheiss, C. Paar, H. Schwab, J. Jose, J. Mol. Catal. B: Enzym. 2002, 18, 89-97.
[38] A. B. Woodside, Z. Huang, C. D. Poulter, Org. Synth. 1988, 66, 211-215.

## Claims

1. A method for displaying a recombinant prenyltransferase on the surface of a host cell, said method comprising the steps:
(a) providing a host cell transformed with a nucleic acid fusion operatively linked with an expression control sequence, said nucleic acid fusion comprising:
(i) a portion encoding a signal peptide,
(ii) a portion encoding the recombinant prenyltransferase to be displayed,
(iii) optionally a portion encoding a protease recognition site,
(iv) a portion encoding a transmembrane linker, and
(v) a portion encoding the transporter domain of an autotransporter,
and
(b) culturing the host cell under conditions wherein the nucleic acid fusion is expressed and the expression product comprising the recombinant prenyltransferase is displayed on the surface of the host cell.

2. The method according to claim 1, wherein the host cell is a bacterium, particularly a gram-negative bacterium, more particularly an enterobacterium, e.g. *E. coli.*

3. The method according to claim 1 or 2, wherein the transporter domain of the autotransporter forms a β-barrel structure.

4. The method according to any one of the preceding claims wherein the transporter domain of the autotransporter is selected from Ssp, Ssp-h1, Ssp-h2, PspA, PspB, Ssa1, SphB1, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protease, App, Hap, rOmpA, rOmpB, ApeE, EstA, Lip-1, McaP, BabA, SabA, AlpA, Aae, NanB, and variants having at least 90% sequence identity thereto, and wherein the transporter domain of the autotransporter preferably is the *E*. *coli* AIDA-I protein or a variant thereof having at least 90% sequence identity to the *E*. *coli* AIDA-I protein.

5. The method according to any one of the preceding claims, wherein the prenyltransferase is selected from isoprenylpyrophosphate synthases, protein prenyltransferases, and aromatic prenyltransferases.

6. The method according to any one of the claims 1 to 5,
(a) wherein the prenyltransferase is selected from isoprenylpyrophosphate synthases catalysing a reaction of a first substrate with a second substrate, said first substrate being selected from isopentenyl pyrophosphate (IPP), dimethyl allyl pyrophosphate (DMAPP), geranyl pyrophosphate (GPP), farnesyl pyrophosphate (FPP), and geranylgeranyl pyrophosphate (GGPP), and said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate, or
(b) wherein the prenyltransferase is selected from protein prenyltransferases catalysing a reaction of a first substrate with a second substrate, said first substrate being selected from proteins, arid said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate, or
(c) wherein the prenyltransferase is selected from aromatic prenyltransferases catalysing a reaction of a first substrate with a second substrate, said first substrate being selected from compounds comprising an aromatic group, and said second substrate being selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), wherein the reaction comprises coupling of the second substrate to the first substrate.

7. A host cell displaying a recombinant polypeptide comprising a prenyltransferase on the surface of the host cell, wherein the recombinant polypeptide comprises
(I) a portion comprising the recombinant prenyltransferase to be displayed,
(II) optionally a portion comprising a protease recognition site,
(III) a portion comprising a transmembrane linker, and
(IV) a portion comprising the transporter domain of an autotransporter.

8. The host cell of claim 7 wherein the recombinant polypeptide is displayed by the transporter domain of an autotransporter.

9. The host cell according to claim 7 or 8 wherein the host cell is a bacterium, particularly a gram-negative bacterium, more particularly an enterobacterium, e.g. *E. coli.*

10. Membrane preparation which is derived from a host cell of any one of the claims 7 to 9, wherein the membrane preparation comprises a recombinant polypeptide comprising
(I) a portion comprising the recombinant prenyltransferase to be displayed,
(II) optionally a portion comprising a protease recognition site,
(III) a portion comprising a transmembrane linker, and
(IV) a portion comprising the transporter domain of an autotransporter,
and wherein the membrane preparation comprises in particular membrane particles.

11. Use of a cell of any one of the claims 7 to 9 or/and a membrane preparation of claim 10
(a) for a prenylation reaction, in particular for the synthesis of organic substances selected from enzyme substrates, drugs, hormones, starting materials and intermediates for synthesis procedures and chiral substances,
(b) for a directed evolution procedure,
(c) as an assay system for a screening procedure,
(d) as a system for toxicity monitoring, or/and
(e) as a system for degrading toxic substances.

12. A method for producing a microorganism displaying a recombinant prenyltransferase on its surface comprising introducing a nucleic acid into said microorganism, said nucleic acid comprising:
(i) a portion encoding a signal peptide,
(ii) a portion encoding the recombinant prenyltransferase to be displayed,
(iii) optionally a portion encoding a protease recognition site,
(iv) a portion encoding a transmembrane linker, and
(v) a portion encoding the transporter domain of an autotransporter,
wherein the nucleic acid is operatively linked with an expression control sequence.

13. A method for prenylation of a first substrate by a prenyltransferase, said method comprising the steps
(a) providing a host cell comprising the prenyl transferase located on its surface, or/and a membrane preparation of the host cell, and
(b) contacting the host cell or/and the membrane preparation thereof with a first substrate and a second substrate, under conditions wherein the prenyltransferase catalyses the reaction of the first substrate and the second substrate, wherein the second substrate is selected from allylic isoprenyl pyrophosphates, and wherein the second substrate is coupled to the first substrate.

14. The method according to claim 13, wherein the prenyltransferase is selected from isoprenylpyrophosphate synthases, protein prenyltransferases, and aromatic prenyltransferases.

15. The method of claim 13 or 14,
(a) wherein the prenyltransferase is selected from isoprenylpyrophosphate synthases, wherein the first substrate is selected from isopentenyl pyrophosphate (IPP), dimethyl allyl pyrophosphate (DMAPP), geranyl pyrophosphate (GPP), farnesyl pyrophosphate (FPP), and geranylgeranyl pyrophosphate (GGPP), and wherein the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), or
(b) wherein the prenyltransferase is selected from protein prenyltransferases, and the first substrate is selected from proteins, and the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP), or
(c) wherein the prenyltransferase is selected from aromatic prenyltransferases, and the first substrate is selected from compounds comprising an aromatic group, and the second substrate is selected from allylic isoprenyl pyrophosphates, such as dimethyl allyl pyrophosphate (DMAPP), geranyl pyrophosphate (GPP), farnesyl pyrophosphate (FPP), and geranylgeranyl pyrophosphate (GGPP).

## Patentansprüche

1. Verfahren zur Präsentation einer rekombinanten Prenyltransferase auf der Oberfläche einer Wirtszelle, wobei das Verfahren die Schritte umfasst:
(a) Bereitstellen einer Wirtszelle transformiert mit einer Nukleinsäurefusion operativ verbunden mit einer Expressionskontrollsequenz, wobei die Nukleinsäurefusion umfasst:
(i) einen Anteil kodierend ein Signalpeptid,
(ii) einen Anteil kodierend die rekombinante Prenyltransferase, welche präsentiert werden soll,
(iii) gegebenenfalls einen Anteil kodierend eine Proteaseerkennungsstelle,
(iv) einen Anteil kodierend einen Transmembranlinker und
(v) einen Anteil kodierend die Transporterdomäne eines Autotransporters, und
(b) Kultivieren der Wirtszelle unter Bedingungen, unter denen die Nukleinsäurefusion exprimiert wird und das Expressionsprodukt umfassend die rekombinante Prenyltransferase auf der Oberfläche der Wirtszelle präsentiert wird.

2. Verfahren gemäß Anspruch 1, wobei die Wirtszelle ein Bakterium, insbesondere ein gramnegatives Bakterium, stärker bevorzugt ein Enterobakterium, z.B. *E. coli*, ist.

3. Verfahren gemäß Anspruch 1 oder 2, wobei die Transporterdomäne des Autotransporters eine β-Fassstruktur bildet.

4. Verfahren gemäß einem der vorhergehenden Ansprüche, wobei die Transporterdomäne des Autotransporters ausgewählt wird aus Ssp, Ssp-h1, Ssp-h2, PspA, PspB, Ssa1, SphB1, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, EspI, EaaA, EaaC, Pertactin, BrkA, Tef, Vag8, PmpD, Pmp20, Pmp21, IgA1-Protease, App, Hap, rOmpA, rOmpB, ApeE, EstA, Lip-1, McaP, BabA, SabA, AlpA, Aae, NanB und Varianten, welche wenigstens 90 % Sequenzidentität dazu haben und wobei die Transporterdomäne des Autotransporters vorzugsweise das *E. coli* AIDA-I Protein oder eine Variante davon mit wenigstens 90 % Sequenzidentität zum *E*. *coli* AIDA-I Protein ist.

5. Verfahren gemäß einem der vorhergehenden Ansprüche, wobei die Prenyltransferase ausgewählt wird aus Isoprenylpyrophosphatsynthasen, Proteinprenyltransferasen und aromatischen Prenyltransferasen.

6. Verfahren gemäß einem der Ansprüche 1 bis 5,
(a) wobei die Prenyltransferase ausgewählt wird aus Isoprenylpyrophosphatsynthasen, welche eine Reaktion eines ersten Substrats mit einem zweiten Substrat katalysieren, wobei das erste Substrat ausgewählt wird aus Isopentenylpyrophosphat (IPP), Dimethylallylpyrophosphat (DMAPP), Geranylpyrophosphat (GPP), Farnesylpyrophosphat (FPP) und Geranylgeranylpyrophosphat (GGPP), und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP), wobei die Reaktion das Kuppeln des zweiten Substrats an das erste Substrat umfasst, oder
(b) wobei die Prenyltransferase ausgewählt wird aus Proteinprenyltransferasen, welche eine Reaktion eines ersten Substrats mit einem zweiten Substrat katalysieren, wobei das erste Substrat ausgewählt wird aus Proteinen, und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP), wobei die Reaktion das Kuppeln des zweiten Substrats an das erste Substrat umfasst, oder
(c) wobei die Prenyltransferase ausgewählt wird aus aromatischen Prenyltransferasen, welche eine Reaktion eines ersten Substrats mit einem zweiten Substrat katalysieren, wobei das erste Substrat ausgewählt wird aus Verbindungen umfassend eine aromatische Gruppe, und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP), wobei die Reaktion das Kuppeln des zweiten Substrats an das erste Substrat umfasst.

7. Wirtszelle präsentierend ein rekombinantes Polypeptid umfassend eine Prenyltransferase auf der Oberfläche der Wirtszelle, wobei das rekombinante Polypeptid umfasst
(I) einen Anteil umfassend die rekombinante Prenyltransferase, welche präsentiert werden soll,
(II) gegebenenfalls einen Anteil umfassend eine Proteaseerkennungsstelle,
(III) ein Anteil umfassend einen Transmembranlinker und
(IV) einen Anteil umfassend die Transporterdomäne eines Autotransporters.

8. Wirtszelle nach Anspruch 7, wobei das rekombinante Polypeptid durch die Autotransporterdomäne eines Autotransporters präsentiert wird.

9. Wirtszelle nach Anspruch 7 oder 8, wobei die Wirtszelle ein Bakterium, insbesondere ein gramnegatives Bakterium, stärker bevorzugt ein Enterobakterium, z.B. *E. coli*, ist.

10. Membranpräparation, welche aus einer Wirtszelle gemäß einem der Ansprüche 7 bis 9 abgeleitet wird, wobei die Membranpräparation ein rekombinantes Polypeptid umfasst, welches umfasst
(I) einen Anteil umfassend die rekombinante Prenyltransferase, welche präsentiert werden soll,
(II) gegebenenfalls einen Anteil umfassend eine Proteaseerkennungsstelle,
(III) ein Anteil umfassend einen Transmembranlinker und
(IV) einen Anteil umfassend die Transporterdomäne eines Autotransporters, und wobei die Membranpräparation insbesondere Membranpartikel umfasst.

11. Verwendung einer Zelle gemäß einem der Ansprüche 7 bis 9 oder/und einer Membranpräparation gemäß Anspruch 10
(a) für eine Prenylierungsreaktion, insbesondere für die Synthese von organischen Substanzen ausgewählt aus Enzymsubstraten, Wirkstoffen, Hormonen, Ausgangsmaterialien und Zwischenprodukten für Syntheseverfahren und chiralen Substanzen,
(b) für ein gerichtetes Evolutionsverfahren,
(c) als ein Testsystem für ein Screeningverfahren,
(d) als ein System zur Toxizitätsüberwachung oder/und
(e) als ein System zum Abbau toxischer Substanzen.

12. Verfahren zur Herstellung eines Mikroorganismus' präsentierend eine rekombinante Prenyltransferase auf seiner Oberfläche, umfassend Einbringen einer Nukleinsäure in den Mikroorganismus, wobei die Nukleinsäure umfasst:
(i) einen Anteil kodierend ein Signalpeptid,
(ii) einen Anteil kodierend die rekombinante Prenyltransferase, welche präsentiert werden soll,
(iii) gegebenenfalls einen Anteil kodierend eine Proteaseerkennungsstelle,
(iv) einen Anteil kodierend einen Transmembranlinker und
(v) einen Anteil kodierend die Transporterdomäne eines Autotransporters,
wobei die Nukleinsäure mit einer Expressionskontrollsequenz operativ verbunden ist.

13. Verfahren zur Prenylierung eines ersten Substrats durch eine Prenyltransferase, wobei das Verfahren die Schritte umfasst
(a) Bereitstellen einer Wirtszelle umfassend die Prenyltransferase, welche auf ihrer Oberfläche lokalisiert ist, oder/und einer Membranpräparation der Wirtszelle und
(b) Inkontaktbringen der Wirtszelle oder/und der Membranpräparation davon mit einem ersten Substrat und einem zweiten Substrat unter Bedingungen, unter denen die Prenyltransferase die Reaktion des ersten Substrats und des zweiten Substrats katalysiert, wobei das zweite Substrat ausgewählt wird aus allylischen Isoprenylpyrophosphaten, und wobei das zweite Substrat an das erste Substrat gekoppelt wird.

14. Verfahren gemäß Anspruch 13, wobei die Prenyltransferase ausgewählt wird aus Isoprenylpyrophosphatsynthasen, Proteinprenyltransferasen und aromatischen Prenyltransferasen.

15. Verfahren gemäß Anspruch 13 oder 14,
(a) wobei die Prenyltransferase ausgewählt wird aus Isoprenylpyrophosphatsynthasen, wobei das erste Substrat ausgewählt wird aus Isopentenylpyrophosphat (IPP), Dimethylallylpyrophosphat (DMAPP), Geranylpyrophosphat (GPP), Farnesylpyrophosphat (FPP) und Geranylgeranylpyrophosphat (GGPP), und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP), oder
(b) wobei die Prenyltransferase ausgewählt wird aus Proteinprenyltransferasen und das erste Substrat ausgewählt wird aus Proteinen und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP), oder
(c) wobei die Prenyltransferase ausgewählt wird aus aromatischen Prenyltransferasen und das erste Substrat ausgewählt wird aus Verbindungen umfassend eine aromatische Gruppe und das zweite Substrat ausgewählt wird aus allylischen Isoprenylpyrophosphaten, wie zum Beispiel Dimethylallylpyrophosphat (DMAPP), Geranylpyrophosphat (GPP), Farnesylpyrophosphat (FPP) und Geranylgeranylpyrophosphat (GGPP).

## Revendications

1. Procédé pour présenter une prényltransférase recombinante sur la surface d'une cellule hôte, ledit procédé comprenant les étapes consistant à :
(a) fournir une cellule hôte transformée avec une fusion d'acide nucléique liée de façon opérationnelle avec une séquence contrôle d'expression, ladite fusion d'acide nucléique comprenant :
(i) une partie codant un peptide signal,
(ii) une partie codant la prényltransférase recombinante devant être présentée,
(iii) éventuellement, une partie codant un site de reconnaissance de protéase,
(iv) une partie codant un lieur transmembranaire, et
(v) une partie codant le domaine transporteur d'un autotransporteur,
et
(b) cultiver la cellule hôte dans des conditions dans lesquelles la fusion d'acide nucléique est exprimée et le produit d'expression comprenant la prényltransférase recombinante est présenté sur la surface de la cellule hôte.

2. Procédé selon la revendication 1, dans lequel la cellule hôte est une bactérie, en particulier une bactérie gram négatif, plus particulièrement une entérobactérie, par exemple *E. coli.*

3. Procédé selon la revendication 1 ou 2, dans lequel le domaine transporteur de l'autotransporteur forme une structure de type tonneau β.

4. Procédé selon l'une quelconque des revendications précédentes, dans lequel le domaine transporteur de l'autotransporteur est choisi parmi Ssp, Ssp-h1, Ssp-h2, PspA, PspB, Ssa1, SphBl, AspA/NalP, VacA, AIDA-I, IcsA, MisL, TibA, Ag43, ShdA, AutA, Tsh, SepA, EspC, EspP, Pet, Pic, SigA, Sat, Vat, EpeA, EatA, EspI, EaaA, EaaC, Pertactine, BrkA, Tef, Vag8, PmpD, Pmp20, Pmp21, protéase d'IgA1, App, Hap, rOmpA, rOmpB, ApeE, EstA, Lip-1, McaP, BabA, SabA, AlpA, Aae, NanB et les variants ayant au moins 90 % d'identité de séquence avec ceux-ci, et dans lequel le domaine transporteur de l'autotransporteur est de préférence la protéine AIDA-I de *E. coli* ou un variant de celle-ci ayant au moins 90 % d'identité de séquence avec la protéine AIDA-I de *E. coli.*

5. Procédé selon l'une quelconque des revendications précédentes, dans lequel la prényltransférase est choisie parmi les isoprénylpyrophosphate synthases, les prényltransférases protéiques et les prényltransférases aromatiques.

6. Procédé selon l'une quelconque des revendications 1 à 5,
(a) dans lequel la prényltransférase est choisie parmi les isoprénylpyrophosphate synthases catalysant une réaction d'un premier substrat avec un second substrat, ledit premier substrat étant choisi parmi l'isopentényl pyrophosphate (IPP), le diméthylallyl pyrophosphate (DMAPP), le géranyl pyrophosphate (GPP), le farnésyl pyrophosphate (FPP) et le géranylgéranyl pyrophosphate (GGPP), et ledit second substrat étant choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP), dans lequel la réaction comprend le couplage du second substrat au premier substrat, ou
(b) dans lequel la prényltransférase est choisie parmi les prényltransférases protéiques catalysant une réaction d'un premier substrat avec un second substrat, ledit premier substrat étant choisi parmi les protéines, et ledit second substrat étant choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP), dans lequel la réaction comprend le couplage du second substrat au premier substrat, ou
(c) dans lequel la prényltransférase est choisie parmi les prényltransférases aromatiques catalysant une réaction d'un premier substrat avec un second substrat, ledit premier substrat étant choisi parmi des composés comprenant un groupe aromatique, et ledit second substrat étant choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP), dans lequel la réaction comprend le couplage du second substrat au premier substrat.

7. Cellule hôte présentant un polypeptide recombinant comprenant une prényltransférase sur la surface de la cellule hôte, dans laquelle le polypeptide recombinant comprend
(I) une partie comprenant la prényltransférase recombinante devant être présentée,
(II) éventuellement, une partie comprenant un site de reconnaissance de protéase,
(III) une partie comprenant un lieur transmembranaire, et
(IV) une partie comprenant le domaine transporteur d'un autotransporteur.

8. Cellule hôte selon la revendication 7, dans laquelle le polypeptide recombinant est présenté par le domaine transporteur d'un autotransporteur.

9. Cellule hôte selon la revendication 7 ou 8, laquelle cellule hôte est une bactérie, en particulier une bactérie gram négatif, plus particulièrement une entérobactérie, par exemple *E. coli.*

10. Préparation membranaire qui est dérivée d'une cellule hôte selon l'une quelconque des revendications 7 à 9, laquelle préparation membranaire comprend un polypeptide recombinant comprenant
(I) une partie comprenant la prényltransférase recombinante devant être présentée,
(II) éventuellement, une partie comprenant un site de reconnaissance de protéase,
(III) une partie comprenant un lieur transmembranaire, et
(IV) une partie comprenant le domaine transporteur d'un autotransporteur,
et laquelle préparation membranaire comprend en particulier des particules membranaires.

11. Utilisation d'une cellule selon l'une quelconque des revendications 7 à 9 ou/et d'une préparation membranaire selon la revendication 10
(a) pour une réaction de prénylation, en particulier pour la synthèse de substances organiques choisies parmi les substrats enzymatiques, les substances médicamenteuses, les hormones, les matières de départ et les produits intermédiaires pour procédés de synthèse et les substances chirales,
(b) pour un mode opératoire d'évolution dirigée,
(c) en tant que système d'essai pour procédé de criblage,
(d) en tant que système pour contrôle de toxicité, ou/et
(e) en tant que système pour dégrader des substances toxiques.

12. Procédé pour produire un micro-organisme présentant une prényltransférase recombinante sur sa surface, comprenant l'introduction d'un acide nucléique dans ledit micro-organisme, ledit acide nucléique comprenant :
(i) une partie codant un peptide signal,
(ii) une partie codant la prényltransférase recombinante devant être présentée,
(iii) éventuellement, une partie codant un site de reconnaissance de protéase,
(iv) une partie codant un lieur transmembranaire, et
(v) une partie codant le domaine transporteur d'un autotransporteur,
dans lequel l'acide nucléique est lié de façon opérationnelle avec une séquence contrôle d'expression.

13. Procédé pour la prénylation d'un premier substrat par une prényltransférase, ledit procédé comprenant les étapes constituant à :
(a) fournir une cellule hôte comprenant la prényltransférase se trouvant sur sa surface, ou/et une préparation membranaire de la cellule hôte, et
(b) mettre en contact la cellule hôte ou/et la préparation membranaire de celle-ci avec un premier substrat et un second substrat, dans des conditions dans lesquelles la prényltransférase catalyse la réaction du premier substrat et du second substrat, dans laquelle le second substrat est choisi parmi les isoprényl pyrophosphates allyliques, et dans laquelle le second substrat est couplé au premier substrat.

14. Procédé selon la revendication 13, dans lequel la prényltransférase est choisie parmi les isoprénylpyrophosphate synthases, les prényltransférases protéiques et les prényltransférases aromatiques.

15. Procédé selon la revendication 13 ou 14,
(a) dans lequel la prényltransférase est choisie parmi les isoprénylpyrophosphate synthases, dans lequel le premier substrat est choisi parmi l'isopentényl pyrophosphate (IPP), le diméthylallyl pyrophosphate (DMAPP), le géranyl pyrophosphate (GPP), le farnésyl pyrophosphate (FPP) et le géranylgéranyl pyrophosphate (GGPP), et dans lequel le second substrat est choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP), ou
(b) dans lequel la prényltransférase est choisie parmi les prényltransférases protéiques, et le premier substrat est choisi parmi les protéines, et le second substrat est choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP), ou
(c) dans lequel la prényltransférase est choisie parmi les prényltransférases aromatiques, et le premier substrat est choisi parmi des composés comprenant un groupe aromatique, et le second substrat est choisi parmi les isoprényl pyrophosphates allyliques, tels que le diméthylallyl pyrophosphate (DMAPP), le géranyl pyrophosphate (GPP), le farnésyl pyrophosphate (FPP) et le géranylgéranyl pyrophosphate (GGPP).
